# EUROPEAN PATENT SPECIFICATION

(11) **EP 1 339 677 B1**
(45) Date of publication and mention of the grant of the patent: **04.01.2006**
(21) Application number: 01976572.6
(22) Date of filing: 26.10.2001
(51) Int. Cl.: C07D 207/09, C07D 409/12, C07D 401/12, C07D 405/12, A61K 31/40, A61P 25/18

(54) **AMINOALKYLPYRROLIDINE SEROTONIN RECEPTOR LIGANDS AND COMPOSITIONS, THEIR PHARMACEUTICAL USES, AND METHODS FOR THEIR SYNTHESIS**
AMINOALKYLPYRROLIDINE ALS SEROTONINREZEPTORLIGANDEN SOWIE DIESE ENTHALTENDE ZUSAMMENSETZUNGEN, PHARMAZEUTISCHE VERWENDUNGEN UND METHODEN ZU DEREN SYNTHESE
LIGANDS DES RECEPTEURS DE SEROTONINE AMINOALKYLPYRROLIDINE ET COMPOSITIONS, LEURS UTILISATIONS PHARMACEUTIQUES, ET PROCEDES POUR LEUR SYNTHESE

(30) Priority: 30.10.2000 US 243710 P
(43) Date of publication of application: 03.09.2003
(73) Proprietor: Warner-Lambert Company LLC, Morris Plains, New Jersey 07950 (US)
(72) Inventor: RUI, Yuanjin, San Diego, CA 92128 (US); KUKI, Atsuo, Encinitas, CA 92024 (US); HONG, Yufeng, San Diego, CA 92128 (US); PENG, Zhengwei, San Diego, CA 92128 (US); LUTHIN, David Robert, Encinitas, CA 92024 (US)
(74) Representative: Hirsch, Denise Marie
(86) International application number: PCT/IB2001/002023
(87) International publication number: WO 2002/036560

(56) References cited:
- WO-A-01/10842
- WO-A-01/55111
- WO-A-99/50264
- FR-A- 1 584 800
- GB-A- 1 552 460
- US-A- 3 721 673
- US-A- 5 037 841
- US-A- 5 571 832
- LOVELL, P. J. ET AL: "A Novel, Potent and Selective 5-HT7 Antagonist: (R)-3-(2-(2-(4-Methylpiperidin-1-yl)-ethyl )pyrrolidine-1-sulfonyl)phenol (SB-269970)" J. MED. CHEM, vol. 43, 21 January 2000 (2000-01-21), pages 342-345, XP002190055 cited in the application
- HAMMER, C. F. ET AL: "Reactions of.beta.-substituted amines. II. Nucleophilic displacement reactions on 3-chloro-1-ethylpiperidine" TETRAHEDRON (1972), 28(2), 239-53 , XP001056797
- SAMULA, KAZIMIERZ ET AL: "4-Pyridinecarboxylic acid derivatives. Part II. Products of reactions with amines and chloramine" POL. J. CHEM. (1983), 57(4-5-6), 573-8 , XP001056818
- KUNJO, A. ET AL: "Studies on Cerebral Protective Agents. III. Novel 4-Arylpyrimidine Derivatives with Anti-anoxic and Anti-lipid Peroxidation Activities (3)" CHEM. PHARM. BULL., vol. 41, no. 1, 1993, pages 139-147, XP001026450
- EBNOETHER A.; JUCKER E.: "Ringerweiterungen bei Reaktionen von 2-[N-Methyl-pyrrolidinyl-(2)]-1-chlor-ätha n und 2-[N-Methyl-piperidyl-(2)]-1-chlor-äthan mit nucleophilen Reagenzien" HELV. CHIM. ACTA, vol. 47, 1964, pages 745-755, XP001056878
- REITSEMA, R. H.: "A Novel Rearrangement of a Piperidine Ring" J. AM. CHEM. SOC., vol. 71, 1949, pages 2041-2043, XP001056881

## Description

### BACKGROUND OF THE INVENTION

### Field of the Invention

The invention relates to aminoalkylpyrrolidine 5-HT₇ receptor ligands, methods of preparing such ligands and intermediates useful in such preparation, and pharmaceutical compositions and treatment methods employing the ligands.

### Description of the Field of the Invention

The neurotransmitter serotonin (5-hydroxytryptamine, or "5-HT") has been the subject of substantial research, and abnormalities in serotonin processing are implicated in diverse disease states. Serotonin exerts its effects mainly in the central nervous, cardiovascular, and gastrointestinal systems through binding to a number of discrete 5-HT receptor types, which are assigned to classes and subclasses, e.g., 5-HT₁, 5-HT_{1A}, 5-HT₃, etc., based on their pharmacological properties such as ligand binding profiles, coupling to second messenger systems, functional activity, and protein structures. The properties, functions, and pharmacology of these receptor subtypes have been reviewed by (a) Kennett, G. A., "Serotonin Receptors and Their Function," TOCRIS Review (http://www.tocris.com/serotonin.htm), published May, 1997; (b) Peroutka, S. J., 1994, "Molecular Biology of Serotonin (5-HT) Receptors, *Synapse* 18, 1,41-260; and (c) Eglen, R. et al., 1997, "The 5-HT₇ Receptor: Orphan Found, TiPs, April 1997 (Vol. 18), pp. 104-107.

While the 5-HT₃ receptor forms a ligand-gated ion channel, most of the other serotonin receptor types are linked to increases or decreases of cyclic AMP production. Receptors of the 5-HT₁ family are negatively coupled to adenylyl cyclase through guanine-nucleotide-binding (G) proteins; those of the 5-HT₂ family stimulate phospholipase C. The 5-HT₄, 5-HT₆, and 5-HT₇ receptors stimulate adenylyl cyclase via Gₛ coupling. Cloning and function of these receptor types are reviewed by Lucas, J. J. and Hen, R., 1995, "New Players in the 5-HT Receptor Field: Genes and Knockouts," TiPS, July, 1995 (Vol. 16) pp. 246-252.

The 5-HT₇ receptors form a distinct family of G-protein coupled receptors positively coupled to adenylyl cyclase. The 5-HT₇ receptor has been cloned from rat, mouse, guinea pig, and human cDNA. Despite a high degree of inter-species homology (95%), the receptor has low homology (<40%) with other 5-HT receptor subtypes. The pharmacological profile of the receptor is also consistent across species and is characterized by a high affinity for the 5-HT₁ agonists, 5-carboxyamidotryptamine (5-CT), 5-HT, and 5-methoxytryptamine.

5-HT₇ receptors are expressed in hypothalamus, hippocampus, thalamus, and other limbic areas and may be involved in regulation of circadian rhythms. 5-HT₇ receptors have high affinity for certain antidepressant and antipsychotic drugs, including pimozide, an antipsychotic used to treat Tourette syndrome, and the atypical antipsychotic drug, clozapine. Biochemical and pharmacologic studies have pointed to the role of 5-HT in the following conditions:
- depression (Sleight, A. J., et al., 1995, "Identification of 5-Hydroxytryptamine₇ Receptor Binding Sites in Rat Hypothalamus: Sensitivity to Chronic Antidepressant Treatment," Molecular Pharmacol. 47:99-103; Shimizu, M. et al., 1996, "Chronic Antidepressant Exposure Enhances 5-Hydroxytryptamine₇ Receptor-Mediated Cyclic Adenosine Monophosphate Accumulation in Rat Frontocortical Astrocytes," J. Pharmacol. Exper. Therapeutics 279:1551-1558);
- psychosis (Roth, B. L. et al., 1994, "Binding of Typical and Atypical Antipsychotic Agents to 5-Hydroxytryptamine-6 and 5-Hydroxytryptamine-7 Receptors," J. Pharmacol. Exper. Therapeutics 268: 1403-1410);
- cardiovascular disease (Cushing, D. J. et al., 1996, "LY215840, a High-Affinity 5-HT₇ Receptor Ligand, Blocks Serotonin-induced Relaxation in Canine Coronary Artery," J. Pharmacol. Exper. Ther. 277:1560-1566; Terron, J., 1998, "The Relaxant 5-HT Receptor in the Dog Coronary Artery Smooth Muscle: Pharmacological Resemblance to the Cloned 5-ht₇ Receptor Subtype," Proc. West. Pharmacol. Soc. 41:129-30); and
- affective behaviors and modulation of sensory information (To, Z. et al., 1995, "Characterization and Distribution of Putative 5-ht₇ Receptors in Guinea Pig Brains," Brit. J. Pharmacol. 115:107-116).

At present, very few selective ligands for 5-HT₇ receptors have been reported (Forbes, L T. et al., "(*R*)-3 N-Dimethyl-N[1-methyl-3(4-methyl-piperidin-1-yl)propyl]benzenesulfonamide: The First Selective 5-HT₇ Receptor Antagonist," *J. Med. Chem.* 41, 655-657 (1998); Kikuchi et al., "Tetrahydrobenzindoles: Selective Antagonists of the 5-HT₇ Receptor," *J. Med. Chern.* 42, 533-535 (1999); Lovell et al., "A Novel Potent, and Selective 5-HT₇ Antagonist: (*R*)-3-(2-(2-(4-Methylpipezidinyl-1-yl)-ethyl)pyrrolidine-1-sulfonyl)phenol (SB-269970)," *J. Med. Chem.* 43, 342-345, (2000); "Functional Characteristics of the Human Cloned 5-HT₇ Receptor (long form) Antagonist Profile of SB-258719," *British J. Pharm,* 124, 1300-1306 (1998); Proos Science (abstracts) of Asai et al., *72*^{*nd*} *Annual Meet Jpn. Pharmacol. Soc.* (March 23-25, Sapporo), 1999 - Abst. P-496, Needham et al., *Eur. Neuropsychopharmacol. [12*^{*th*} *Cong. Eur. Coll. Neuropsychopharmacol. (Sept. 21-25, London)]* 1999, 9, (Suppl.5) - Abst. P.2.021; WO 99/31062 and WO/00/0472).

WO 01/10842 discloses MC4 receptor binding compounds of formula B-L₁-A-L₂-E wherein B is an anchor moiety, L₁ and L₂ are linking moiety, A is a cyclic moiety and E is a MC4-R interacting moiety. US 5037841 discloses 1,3-disubstituted pyrrolidines active on the central nervous system of the formula

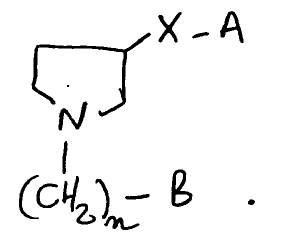

Tetrahedron. Vol 28, *pp*. 239 to 253 discloses compounds of formula 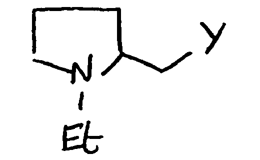 wherein Y may be -N(CH₂-Ph)₂.

The 5-HT₇ receptor may be involved in the pathophysiology of sleep disorders, depression, pain, and schizophrenia. Potent and selective ligands active at 5-HT₇ receptors are needed to provide novel pharmaceutical approaches to treatment of these disorders.

### SUMMARY OF THE INVENTION

This invention is directed to compounds represented by Formula I: 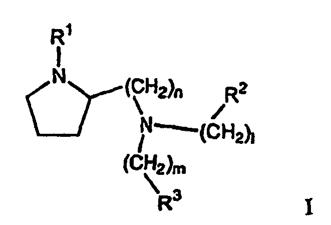
wherein:
l, m, and n are independently 1 or 2;
R¹ is C₁-C₆ alkyl;
R² and R³ may be the same or different and are independently selected from substituted or unsubstituted aryl, heteroaryl, arylalkyl, heteroarylalkyl, and cycloalkenyl, provided that when R¹ is ethyl and 1, m and n are each 1, R² and R³ are not both unsubstituted phenyl with the exception of the following compounds -(3-bromo-benzyl)-(1-ethyl-pyrrolidin-2-ylmethyl)-naphtalen-1-ylmethyl-amine, -(5-bromo-2-methoxy-benzyl)-(1-ethyl-pyrrolidin-2-ylmethyl)-naphtalen-1-ylmethyl-amine-(1-ethyl-pyrrolidin-2-ylmethyl)-naphtalen-2-ylmethyl-naphtalen-1-ylmethyl-amine. . These compounds are potent antagonists for 5-HT₇ receptors and show selectivity for 5-HT₇ receptors over other serotonin receptor subtypes and over other receptors such as D₂ dopamine, α₁ adrenergic (α_{1A}, α_{1B}, α_{1D}), α₂ adrenergic (α_{2A}, α_{2B}, α_{2C}), bGalanin, opiate (δ, µ, κ), GABA-B, and muscarinic (M₁, M₂, M₃, M₄, M₅). The compounds have potential utility in the treatment of pain, depression, sleep disorders, and schizophrenia.

The invention also encompasses pharmaceutically acceptable salts or solvates, comprising the compounds of Formula I, and includes pharmaceutical compositions comprising the compounds of Formula I as well as pharmaceutically acceptable salts or solvates thereof. The invention is also related to the use of a compound of Formula I, or a pharmaceutically acceptable salt or, solvate, thereof as a medicament. The invention is also directed to methods of preparation of the compounds represented by Formula I by reductive amination of aminoalkylpyrrolidines with aldehydes. The invention also comprises intermediates and pharmaceutically acceptable salts thereof, useful in the synthesis of compounds of Formula I.

### DETAILED DESCRIPTION OF THE INVENTION

As used herein, the term "alkyl" represents a straight- or branched-chain saturated hydrocarbon group, containing 1 to 20 carbon atoms Exemplary alkyl groups include, but are not limited to methyl (Me), ethyl (Et), propyl, isopropyl, butyl, isobutyl, t-butyl, and the like. The term " lower alkyl" refers to an alkyl group having from 1 to 6 carbon atoms in its chain.

"Cycloalkyl" represents a group comprising a saturated monocyclic, bicyclic, or tricyclic hydrocarbon containing from 3 to 14 carbon atoms that may be a mono- or poly-carbocyclic ring, preferably having 5-14 ring carbon atoms. Exemplary cycloalkyl groups include monocyclic rings having from 3-7, preferably 3-6, carbon atoms, such as cyclopropyl, cyclobutyl, cyclopentyl. cyclohexyl, cyclobeptyl and the like. Exemplary bicyclic and tricyclic cycloalkyls include groups having from 10-14 carbon atoms. Illustrative examples of cycloalkyl groups include the following:

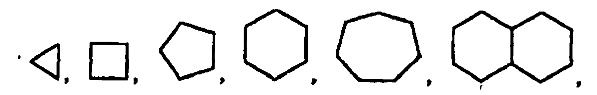

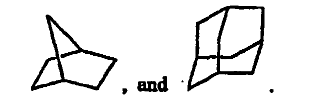

"Cycloalkenyl" represents a group comprising a partially saturated, non-aromatic monocyclic, bicyclic, or tricyclic hydrocarbon containing from 3 to 14 carbon atoms that may be a mono- or poly-carbocyclic ring, preferably having 5-14 ring carbon atoms. Exemplary cycloalkenyl groups include monocyclic rings having from 3-7, preferably 3-6, carbon atoms, such as cyclopentenyl, cyclopentadienyl, cyclohexenyl, cycloheptenyl and the like. Illustrative examples of cycloalkenyl groups include the following:

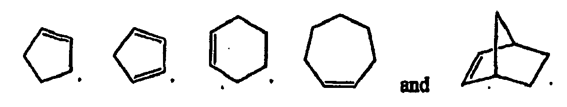

"Heterocycloalkyl" represents a group comprising a non-aromatic, monovalent monocyclic, bicyclic, or tricyclic radical, which is saturated or partially unsaturated, containing 3 to 18 ring atoms, which includes 1 to 5 heteroatoms selected from nitrogen, oxygen and sulfur, and which may be unsubstituted or substituted by one or more of the substituents described below. Illustrative examples of heterocycloalkyl groups include, but are not limited to, azetidinyl, pyrrolidyl, piperidyl, piperazinyl, morpholinyl, tetrahydro-2H-1,4-thiazinyl, tetrahydrofuryl, dibydrofuryl, tetrahydropyranyl, dibydropyranyl, 1,3-dioxolanyl, 1,3-dioxanyl, 1,4-dioxanyl, 1,3-oxathiolanyl, 1,3-oxathianyl, 1,3-dithianyl, azabicylo[3.2.1]octyl, azabicylo[3.3.1]nonyl, azabicylo[4.3.0]nonyl, oxabicylo[2.2.1]heptyl, 1,5,9-triazacyclododecyl, and the like. Illustrative examples of heterocyclodkyl groups include the following moieties:

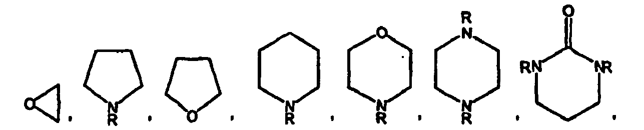

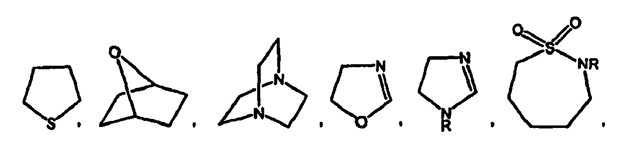

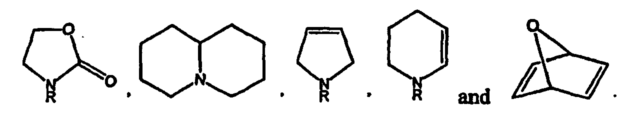

"Aryl" represents a group comprising an aromatic, monovalent monocyclic, bicyclic, or tricyclic radical containing from 6 to 18 carbon ring atoms, which may be unsubstituted or substituted by one or more of the substituents described below. Illustrative examples of aryl groups include the following:

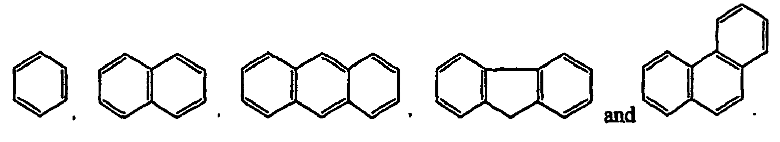

"Heteroaryl" represents a group comprising an aromatic monovalent monocyclic, bicyclic, or tricyclic radical. containing 5 to 18 ring atoms, including 1 to 5 heteroatoms selected from nitrogen, oxygen and sulfur, which may be unsubstituted or substituted by one or more of the substituents described below. Illustrative examples of heteroaryl groups include, but are not limited to, thienyl, pyrrolyl, imidazolyl, pyrazolyl, furyl, isothiazolyl, furazanyl, isoxazolyl, thiazolyl, pyridyl, pyrazinyl, pyrimidinyl, pyridazinyl, triazinyl, benzo[b]thienyl, naphtho[2,3-b]thianthrenyl, isobenzofuranyl, chromenyl, xanthenyl, phenoxathienyl, indolizinyl, isoindolyl, indolyl, indazolyl, purinyl, isoquinolyl, quinolyl, phthalazinyl, naphthyridinyl, quinoxyalinyl, quinzolinyl, benzothiazolyl, benzimidazolyl, tetrahydroquinolinyl, cinnolinyl, pteridinyl, carbazolyl, beta-carbolinyl, phenanthridinyl, acridinyl, perimidinyl, phenanthrolinyl, phenazinyl, isothiazolyl, phenothiazinyl, and phenoxazinyl. Further examples of heteroaryl groups include the following moieties:

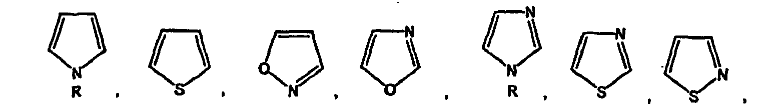

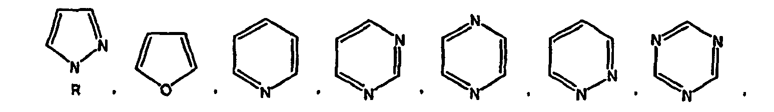

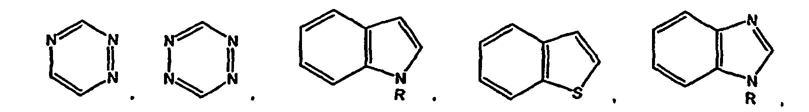

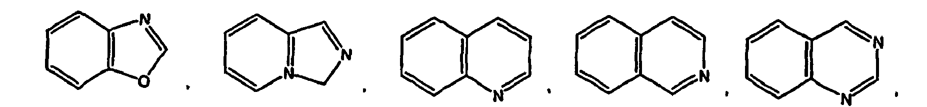

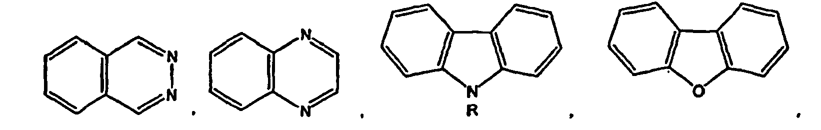

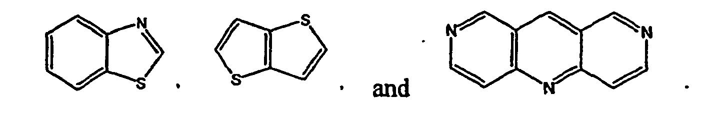

As indicated herein, the alkyl, cycloalkyl, aryl, heterocycloalkyl and heteroaryl groups may be optionally substituted by one or more substituents. The term "optionally substituted" is intended to expressly indicate that the specified group is unsubstituted or substituted by one or more suitable substituents. The term "substituent" or "suitable substituent" is intended to mean any suitable substituent that may be recognized or selected, such as through routine testing, by those skilled in the art.

Exemplary "suitable substituents" that may be present on any of the above alkyl, aryl, cycloalkyl, heterocycloalkyl or heteroaryl groups are described herein and include alkyl (except for alkyl), aryl, cycloalkyl, heterocycloalkyl, heteroaryl, nitro, amino, cyano, halo, hydroxyl, alkoxy, alkylenedioxy, aryloxy, cycloalkoxy, heterocycloallcoxy, heteroaryloxy, alkylcarbonyl, alkyloxycarbonyl, alkylcarbonyloxy, arylcarbonyl, arylcarbonyloxy, aryloxycarbonyl, cycloalkylcarbonyl, cycloalkylcarbonyloxy, cycloalkyoxycarbonyl, heteroarylcarbonyl, heteroarylcarbonyloxy, heteroaryloxycarbonyl, heterocycloalkylcarbonyl, heterocycloalkylcarbonyloxy, heterocycloalkyoxycarbonyl, carboxyl, carbamoyl, formyl, keto (oxo), thioketo, sulfo, alkylamino, cycloalkylamino, arylamino, heterocycloalkylamino, heteroarylamino, dialkylamino, alkylaminocarbonyl, cycloalkylaminocarbonyl, arylaminocarbonyl, heterocycloalkylaminocarbonyl, heteroarylaminocarbonyl, dialkylaminocarbonyl, alkylaminothiocarbonyl, cycloalkylaminothiocarbonyl, arylaminothiocarbonyl, heterocycloalkylaminothiocarbonyl, heteroarylaminothiocarbonyl, dialkylaminothiocarbonyl, alkylsulfonyl, arylsulfonyl, alkylsulfenyl, arylsulfenyl, alkylcarbonylamino, cycloalkylcarbonylamino, arylcarbonylamino, heterocycloalkylcarbonylamino, heteroarylcarbonylamino, alkylthiocarbonylamino, cycloalkylthiocarbonylamino, mylthiocarbonylamino, heterocycloalkylthiocarbonylamino, heteroarylthiocarbonylamino, alkylsulfonyloxy, arylsulfonyloxy, alkylsulfonylamino, arylsulfonylamino, mercapto, alkylthio, arylthio, heteroarylthio, wherein any of the alkyl, alkylene, aryl, cycloalkyl, heterocycloalkyl, heteroaryl moieties present in the above substituents may be further substituted. The alkyl, alkylene, cycloalkyl, heterocycloalkyl, aryl, and heteroaryl moieties of any of the above substituents may be optionally substituted by one or more of alkyl (except for alkyl), haloalkyl, aryl, nitro, amino, alkylamino, dialkylamino, halo, hydroxyl, alkoxy, haloalkoxy, aryloxy, mercapto, alkylthio or arylthio groups.

Preferred "suitable substituents" in the compounds of this invention include lower alkyl, substituted or unsubstituted aryl, arylalkyl, heteroarylalkyl, cycloalkyl, heterocycloalkyl, heteroaryl, halo, hydroxyl, alkoxy, aryloxy, cycloalkoxy, heteroaryloxy, nitro, alkylthio, arylthio and aminocarboxyl.

The terms "halogen" and "halo" represent chloro, fluoro, bromo or iodo substituents. "Heterocycle" is intended to mean a heteroaryl or heterocycloalkyl group. "Acyl" is intended to mean a -C(O)-R radical, wherein R is an alkyl, cycloalkyl, aryl, heterocycloalkyl or heteroaryl group. "Acyloxy" is intended to mean an -OC(O)-R radical, wherein R is an alkyl, cycloallcyl, aryl, heterocycloalkyl or heteroaryl group. ' "Thioacyl" is intended to mean a -C(S)-R radical, wherein R is an alkyl cycloalkyl, aryl, heterocycloalkyl or heteroaryl group "Sulfonyl" is intended to mean an -SO₂- biradical. "Sulfenyl" is intended to mean an -SO- biradical." Sulfo" is intended to mean an -SO₂H radical. "Hydroxy" is intended to mean the radical -OH.) "Amine" or "amino" is intended to mean the radical NH₂. "Alkylamino" is intended to mean the radical -NHRₐ, wherein Rₐ is an alkyl group." Dialkylamino" is intended to mean the radical -NRₐR_{b}, wherein Rₐ and R_{b} are each independently an alkyl group, and is intended to include heterocycloalkyl groups, wherein Rₐ and R_{b}, taken together, form a heterocyclic ring that includes the amine nitrogen. "Alkoxy" is intended to mean the radical -ORₐ, wherein Rₐ is an alkyl group. Exemplary alkoxy groups include methoxy, ethoxy, propoxy, and the like". Lower alkoxy" groups have alkyl moieties having from 1 to 4 carbons. "Alkylenedioxy" is intended to mean the divalent radical -ORₐO- which is bonded to adjacent atoms (e.g., adjacent atoms on a phenyl or naphthyl ring) , wherein Rₐ is a lower alkyl group. "Alkoxycarbonyl" is intended to mean the radical -C(O)ORₐ, wherein Rₐ is an alkyl group. "Alkylsulfonyl" is intended to mean the radical -SO₂Rₐ, wherein Rₐ is an alkyl group. "Alkylaminocarbonyl" is intended to mean the radical -C(O)NHRₐ, wherein Rₐ is an alkyl group. "Dialkylaminocarbonyl" is intended to mean the radical -C(O)NRₐR_{b}, wherein Rₐ and R_{b} are each independently an alkyl group. "Mercapto" is intended to mean the radical -SH. "Alkylthio" is intended to mean the radical -SRₐ, wherein Rₐ is an alkyl group. "Carboxyl" is intended to mean the radical -C(O)OH. "Keto" or "oxo" is intended to mean the radical =O. "Thioketo" is intended to mean the radical =S. "Carbamoyl" is intended to mean the radical -C(O)NH₂. "Cycloalkylalkyl" is intended to mean the radical Balkyl-cycloalkyl, wherein alkyl and cycloalkyl are defined as above, and is represented by the bonding arrangement present in the groups -CH₂-cyclohexane or -CH₂-cyclohexene. "Arylalkyl" is intended to mean the radical alkylaryl, wherein the alkyl and aryl moieties thereof are defined as above (e.g., wherein "alkyl" represents a shaight- or branched-chain saturated hydrocarbon group, containing 1 to 20 carbon atoms, which may be unsubstituted or substituted by one or more substituents) and is represented by the bonding arrangement present in a benzyl group. "Heteroarylalkyl" is intended to mean the radical alkyl-heteroaryl, wherein the alkyl and heteroaryl moieties thereof are defined as above and is represented by the bonding arrangement present in an α-methylfuranyl group. "Aminocarbonylalkyl" is intended to mean the radical alkylC(O) NH₂ and is represented by the bonding arrangement present in the group -CH₂CH₂C(O)NH₂. "Alkylaminocarbonylalkyl" is intended to mean the radical alkylC(O)NHR_{a,} wherein Rₐ is an alkyl group and is represented by the bonding arrangement present in the group -CH₂CH₂C(O)NHCH₃. "Alkylcarbonylaminoalkyl" is intended to mean the radical alkylNHC(O)-alkyl and is represented by the bonding arrangement present in the group -CH₂NHC(O)CH₃. "Dialkylaminocarbonylalkyl" is intended to mean the radical alkylC(O)NRₐR_{b}, wherein Rₐ and R_{b} are each independently an alkyl group. "Aryloxy" is intended to mean the radical -OR_{c}, wherein R_{c} is an aryl group. "Heteroaryloxy" is intended to mean the radical -OR_{d}, wherein R_{d} is a heteroaryl group. "Arylthio" is intended to mean the radical -SR_{c}, wherein R_{c} is an aryl group. "Heteroarylthio" is intended to mean the radical -SR_{d}, wherein R_{d} is a heteroaryl group.

If the substituents themselves are not compatible with the synthetic methods of this invention, the substituent may be protected with a suitable protecting group that is stable to the reaction conditions used in these methods. The protecting group may be removed at a suitable point in the reaction sequence of the method to provide a desired intennediate or target compound. Suitable protecting groups and the methods for protecting and de-protecting different substituents using such suitable protecting groups are well known to those skilled in the art; examples of which may be found in T. Greene and P. Wuts, Protecting Groups in Chemical Synthesis (3rd ed.), John Wiley & Sons, NY (1999), which is incorporated herein by reference in its entirety. In some instances, a substituent may be specifically selected to be reactive under the reaction conditions used in the methods of this invention. Under these circumstances, the reaction conditions convert the selected substituent into another substituent that is either useful in an intermediate compound in the methods of this invention or is a desired substituent in a target compound.

If an inventive compound is a base, a desired salt may be prepared by any suitable method known in the art, including treatment of the free base with an inorganic acid, such as hydrochloric acid, hydrobromic acid, sulfuric acid, nitric acid, phosphoric acid, and the like, or with an organic acid, such as acetic acid, maleic acid, succinic acid, mandelic acid, fumaric acid, malonic acid, pyruvic acid, oxalic acid, glycolic acid, salicylic acid, pyranosidyl acid, such as glucuronic acid or galacturonic acid, alpha-hydroxy acid, such as citric acid or tartaric acid, amino acid, such as aspartic acid or glutamic acid, aromatic acid, such as benzoic acid or cinnamic acid, sulfonic acid, such as p-toluenesulfonic acid or ethanesulfonic acid, or the like.

The inventive compounds may exist as single stereoisomers and/or diastereomers, racemates, and/or mixtures of enantiomers and/or diastereomers. All such single stereoisomers, diastereomers, racemates, and mixtures thereof are intended to be encompassed within the broad scope of the present invention. Where the stereochemistry of the chiral carbons present in the chemical structures illustrated herein is not specified, the chemical structure is intended to encompass compounds containing either stereoisomer of each chiral carbon. Preferably, however, the inventive compounds are used in optically pure form. When used describe a particular compound, the term "optically pure" is used herein to that the compound is substantially enantiomerically or diastereomerically pure. Compounds that are substantially enatiomerically pure contain at least 90% of a single isomer and preferably contain at least 95% of a single isomer. Compounds that are substantially diastereomerically pure contain at least 90% of a single isomer of each chiral carbon center present in the diastereomer, and preferably contain at least 95% of a single isomer of each chiral carbon. More preferably, the optically active compounds in this invention contain at least 97.5% of a single isomer and most preferably contain at least 99% of a single isomer. Compounds identified herein as single stereoisomers are meant to describe compounds that are present in a form that contains at least 90% of a single isomer. The term "racemic" or "racemic mixture" refers to a mixture of equal amounts of enantiomeric compounds, which encompasses mixtures of enantiomers and mixtures of enantiomeric diastereomers.

Preferred embodiments of the compounds of this invention are represented by the Formula: 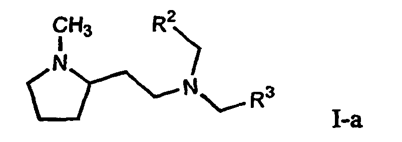
wherein R² and R³ are as defined above. Exemplary R² and R³ groups include, but are not limited to substituted or unsubstituted benzyl, methyldibenzofuranyl, cyclohexenyl, fluorenyl, phenyl, naphthyl, furanyl, benzofuranyl, benzothienyl, dibenzofuranyl and the like, wherein any alkyl (-CH₂-), alkenyl (-CH=) or aryl (-CH=) moiety thereof may be independently substituted by one or more suitable substitutents. Exemplary groups suitable as substitutents for the above-described substituted R² and R³ groups (e.g., substituted benzyl, etc.) include, but are not limited to, lower alkyl, substituted or unsubstituted aryl, arylalkyl, heteroarylalkyl, cycloalkyl, heterocycloalkyl, heteroaryl, halo, hydroxyl, alkoxy, aryloxy, cycloalkoxy, heteroaryloxy, nitro, alkylthio, arylthio and aminocarboxyl.

In especially preferred embodiments, the absolute stereochemistry at the point of attachment of the side-chain to the pyrrolidine ring is as shown in Formula I-b:

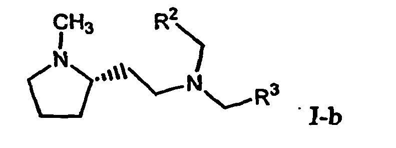

Preferably, in the compounds of this invention represented by Formulas I, I-a, or I-b, R² and R³ are substituted phenyl which may be independently substituted by one or more lower alkyl, halo, hydroxyl, alkoxy, nitro, alkylthio, or aminocarbonyl.

This invention also encompasses methods for preparing the compounds shown above. The compounds may be prepared by reductive amination comprising treatment of an aminoalkylpyrrolidine with aldehydes, under conventional reducing conditions. The reductive amination reactions may be conducted in a stepwise manner, for example: 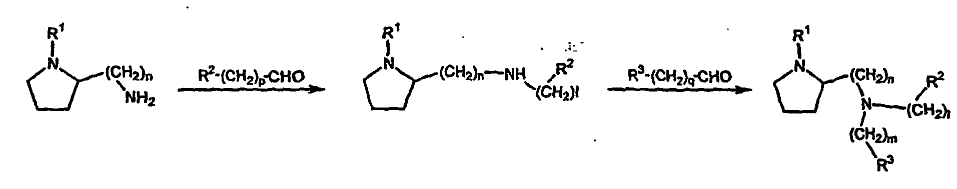 where I, m, R¹, R², and R³ have the meanings given above, p is equal to (1-1), and q is equal to (m-1). However, when R² and R³ are the same, the reductive amination may be conducted in a single step:

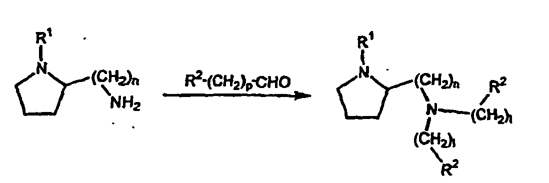

The compounds may also be prepared by amino-alkylation comprising treatment of the aminoalkylpyrrolidine with a suitable alkylating agent under conventional conditions. Symmetrically substituted compounds, wherein the added substituents on nitrogen are the same, may be prepared by conducting the alkylation of the aminoalkylpyrrolidine in a single step: 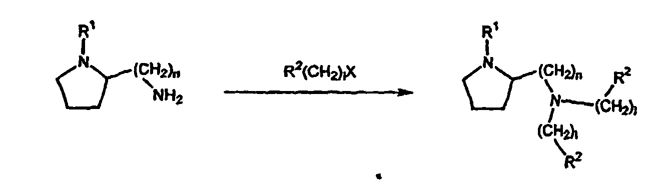
where 1, n, R¹ and R² have the meanings given above and X is a suitable halogen or leaving group. Unsymmetrically substituted compounds may be prepared by treatment of an alkylated aminoalkylpyrrolidine (e.g., which may be prepared as described above by reductive amination of an aminoalkylpyrrolidine) with a suitable alkylating agent, for example: 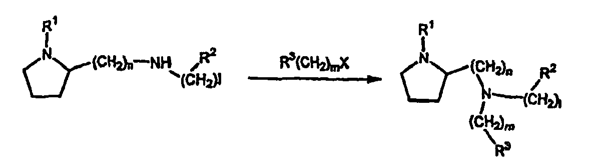
where 1, m, n, R¹, R², and R³ have the meanings given above, and X is a suitable halogen or leaving group.

The invention is also directed to intermediate aminoalkylpyrrolidine compounds and pharmaceutically acceptable salts thereof which are useful in the synthesis of compounds of Formula I.

The aminoalkylpyrrolidine intermediates used therein may be prepared by the following general method :

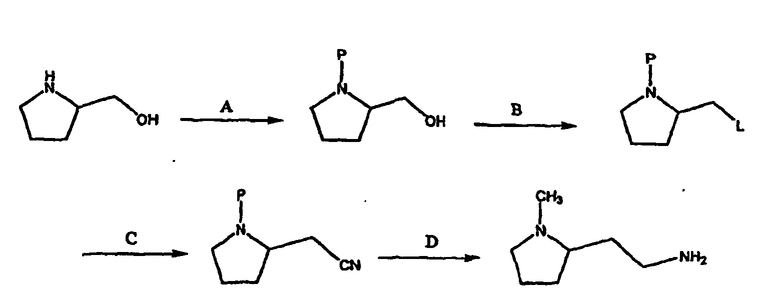

In the first step (Step A) of this method, a hydroxyalkylpyrrolidine is N-protected with protecting group "P" using conventional techniques. The hydroxy moiety is converted in Step B into a leaving group. Suitable leaving groups include tosylate, mesylate, triflate, halo, and the like. The conversion of the hydroxyl moiety into these suitable leaving groups may be conducted using conventional procedures. The leaving group may be displaced in Step C using sodium cyanide, or another suitable a nitrogen-containing nucleophilic reagent to provide a cyanoalkylpyrrolidine. Reduction of the cyano moiety provides the aminoalkylpyrrolidine useful in the method of this invention. Preferably, the protecting group is selected such that during Step D, the protecting group is converted to a suitable R¹ group. Alternatively, R¹ may be directly introduced in Step A or may be introduced during Step D (wherein Step D may comprise two or more steps to affect removal of the protecting group, introduction of R¹ and reduction of the cyano moiety). Alternatively, compounds and intermediates having varying spacer lengths (n is 1 or 2) may be prepared by the following general method: 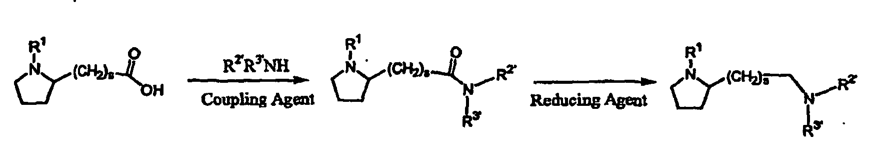
wherein is 0 or 1, R^{2'} and R^{3'} represent -(CH₂)-R² and -(CH₂)-R³, respectively, R¹ is defined as above or is a precursor or protecting group that can be converted to a lower alkyl group during reaction with a reducing agent

In preferred embodiments of this invention, the intermediate compounds, or a pharmaceutically acceptable salt thereof, possess a structure that may be represented by Formula II-a: 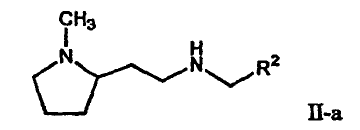
wherein R² is selected from aryl, heteroaryl, arylalkyl, heteroarylalkyl and cycloakenyl each optionally substituted by one or more substituents with the exception of the following compounds : -(2,5-dimethyl-1-phenyl-1H-pyrrol-3-ylmethyl)-[2-(1-methyl-pyrrolidin-2-yl)-ethyl] amine, and, -benzyl-[2-(1-methyl-pyrrolidin-2-yl)-ethyl]-amine.

In especially preferred embodiments, the intermediates compounds, or a pharmaceutically acceptable salt thereof, have Formula II-b: 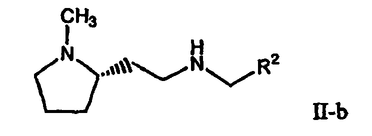
wherein R² is defined as above.

Exemplary compounds of the invention include: 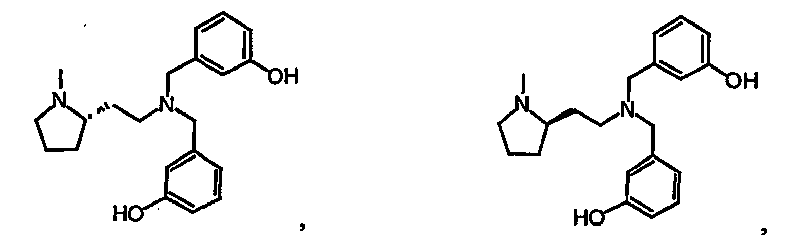 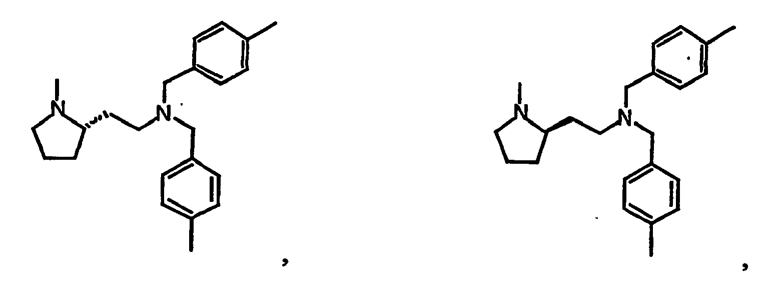 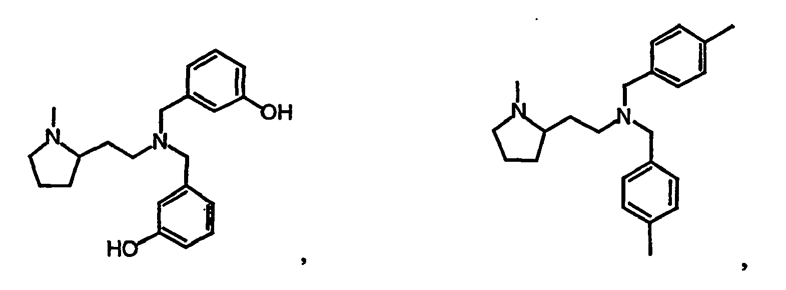 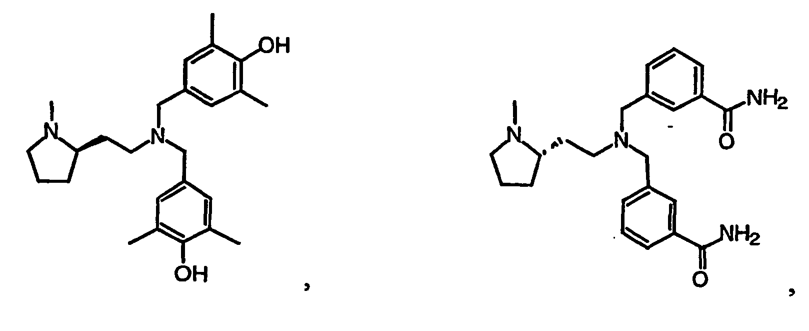 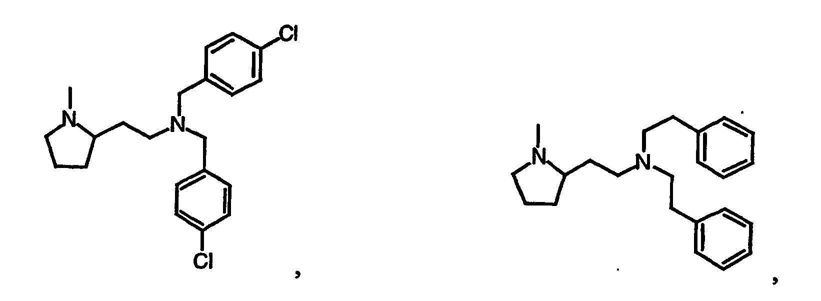 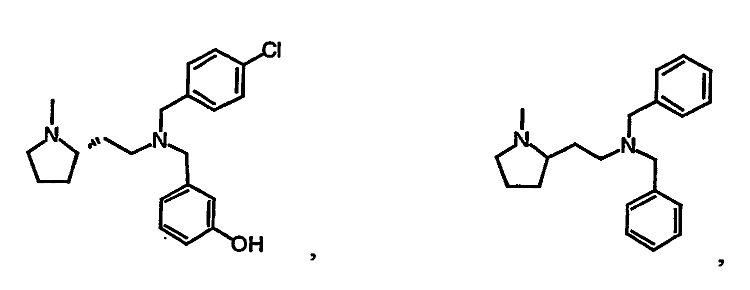 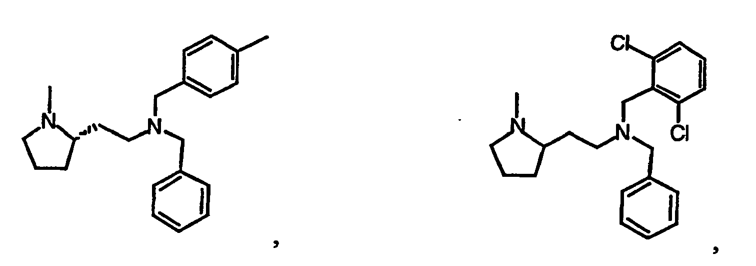 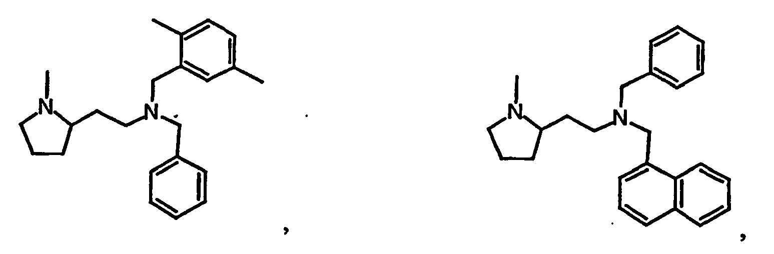 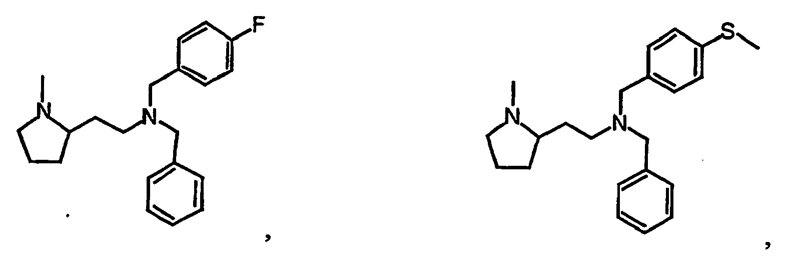 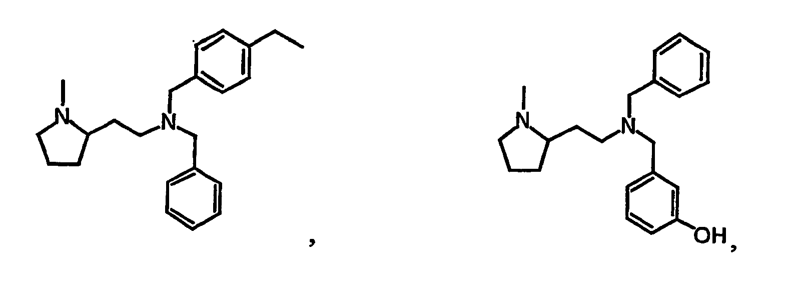 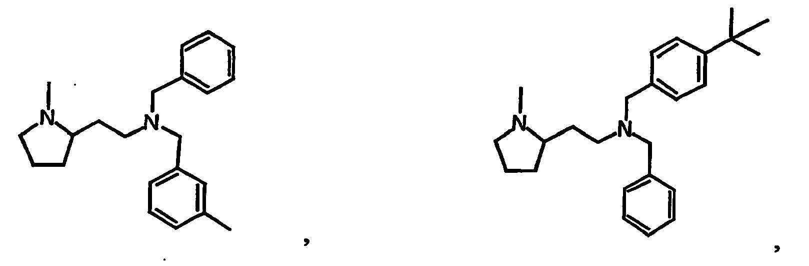 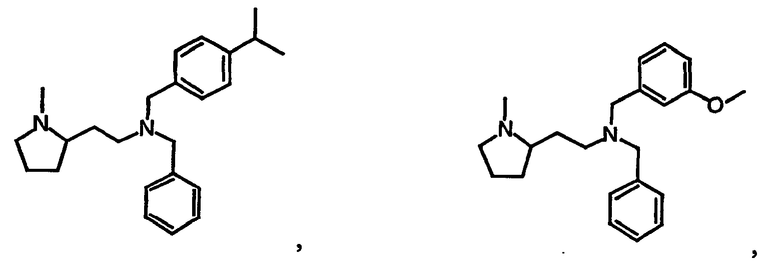 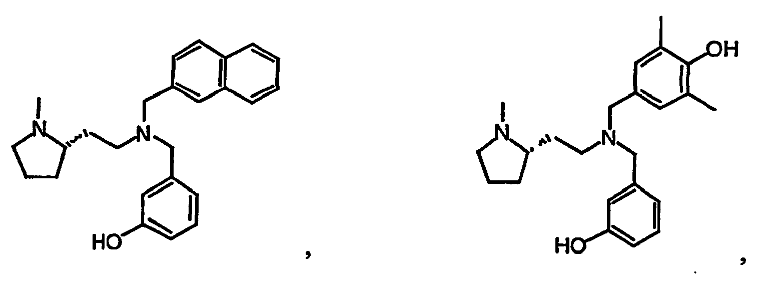 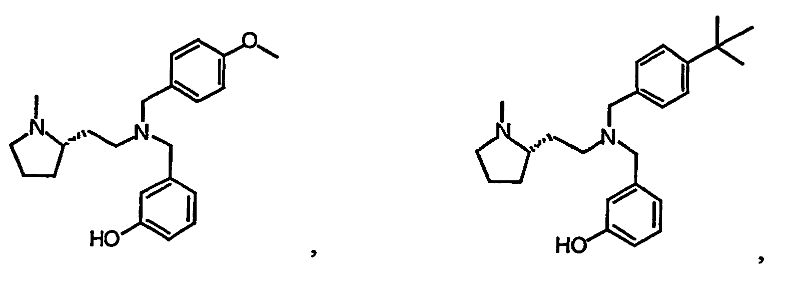 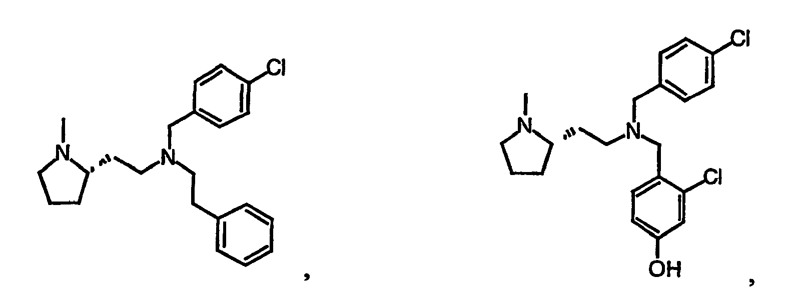 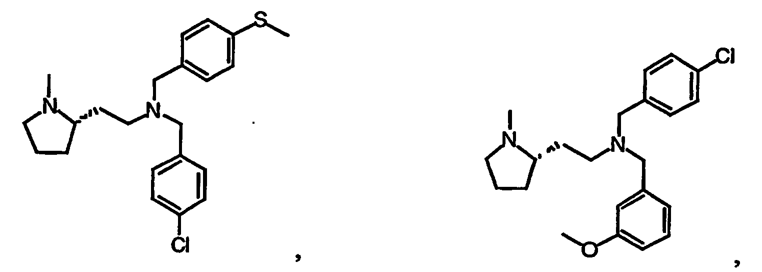 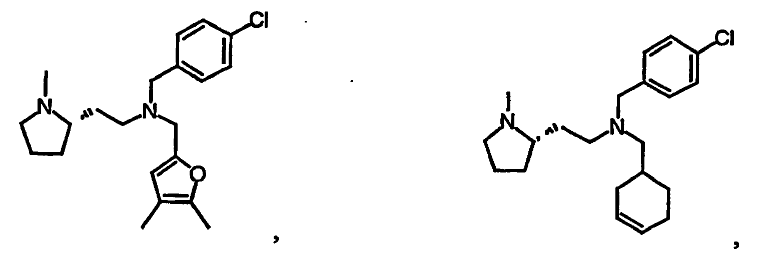 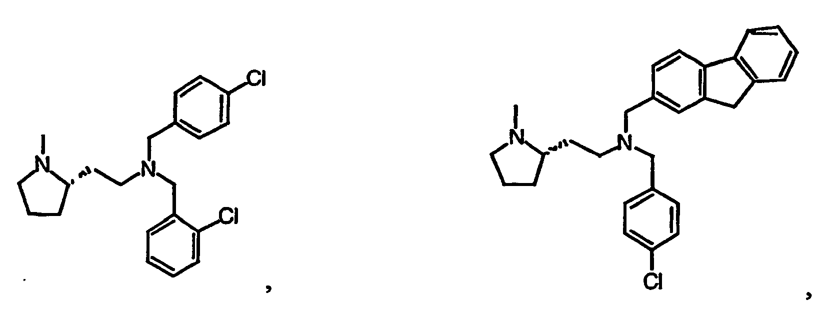 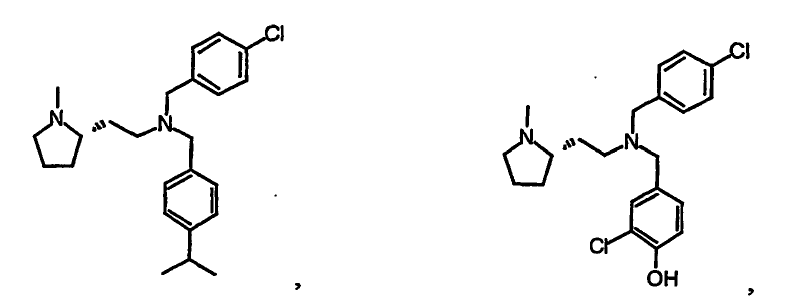 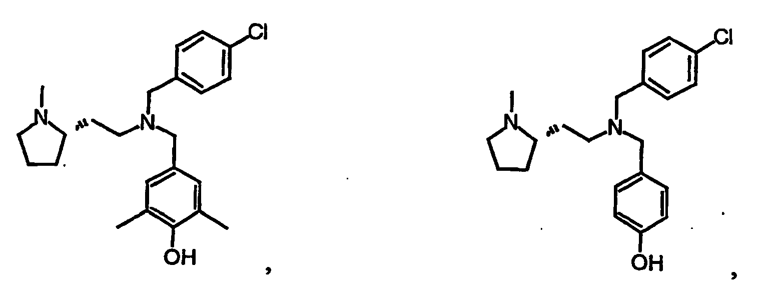 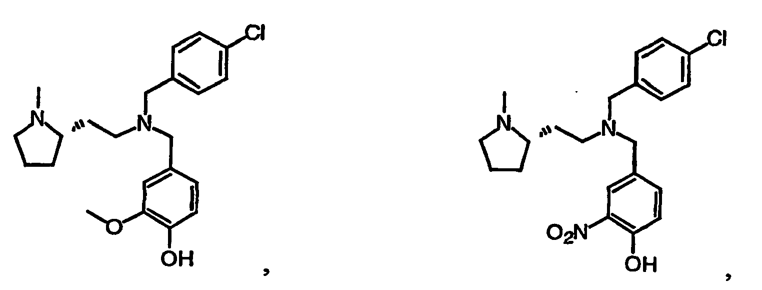 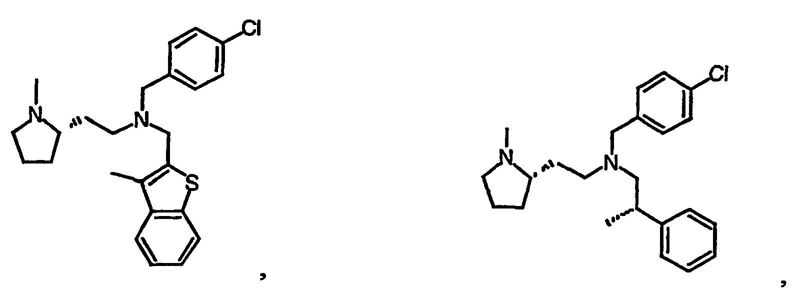 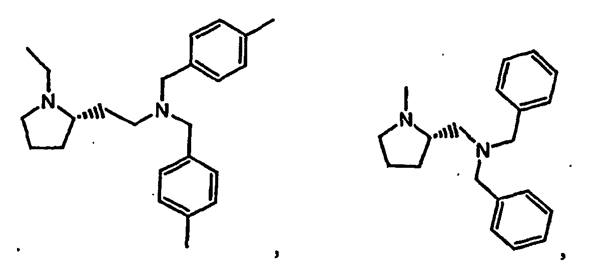 and the pharmaceutically acceptable salts, solvates, active metabolites, or prodrugs thereof.

Particularly preferred compounds of this invention include: 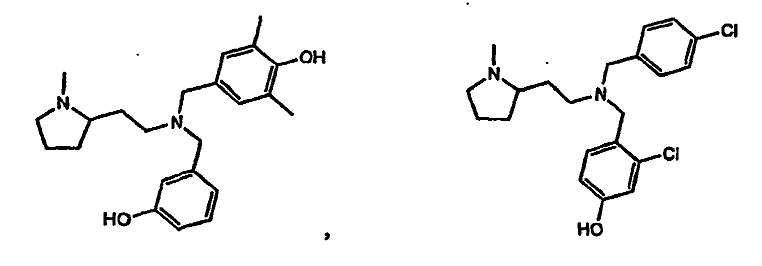 and the pharmaceutically acceptable salts, solvates, active metabolites, or prodrugs thereof.

Exemplary intermediate compounds useful in the preparation of the compounds of this invention include: 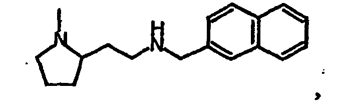 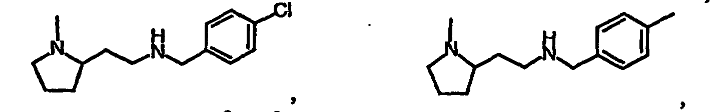 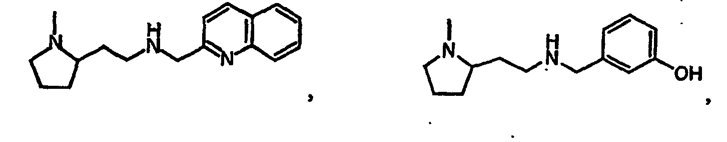 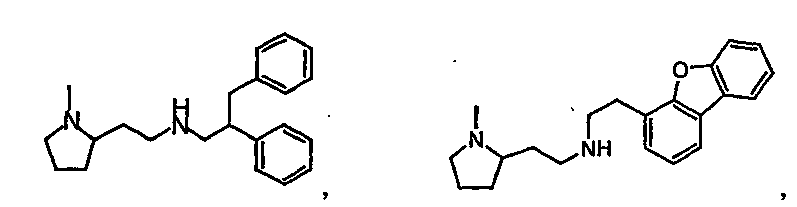 and pharmaceutically acceptable salts thereof.

Particularly preferred intermediate compounds useful in the preparation of the compounds of this invention include: 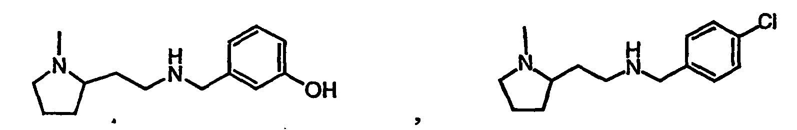 and pharmaceutically acceptable salts thereof.

The compounds of the invention interact with 5-HT receptors and show selectivity for 5-HT receptors. The 5-HT receptor binding properties of the compounds are identified by competitive radioligand binding assays wherein membranes prepared from transfected cells expressing the 5-HT receptor subtype of interest. "Binding constants" refers herein to Kᵢ values measured by inhibition of the binding of radiolabelled ligands that are selective for the 5-HT receptor type being studied. For 5-HT₇ receptors, Kᵢ values are determined by measuring the inhibition of 5-carboxamidotryptamine (5-CT) binding, wherein 5-HT₇ receptors were incubated with the radiolabelled high affinity ligand, 5-carboxamidotryptamine ([³H]5-CT), in the presence and absence of the compounds of the invention, at varying concentrations.

The compounds of the invention have high binding affinity for serotonin receptors as measured by dissociation constant Kᵢ. The compounds of the present invention preferably show 5-HT₇ receptor binding characterized by Kᵢ values less than about 100 nM, more preferably by Kᵢ values less than about 10 nM, and most preferably by Kᵢ values less than about 1 nM. "Selectivity" for receptor type, in the context of this invention, refers to the ratio of binding constants for the two receptor types being compared. For example, if a hypothetical ligand shows Kᵢ of 100 nM for 5-HT₄ receptors and 0.5 nM for 5-HT₇ receptors, its selectivity for 5-HT₇ over 5-HT₄ receptors is 200-fold. The compounds of the present invention preferably show selectivity for 5-HT₇ receptors over other serotonin receptor subtypes of greater than about 100. The compounds of the present invention also preferably show selectivity for 5-HT₇ receptors over other receptor types, such as dopamine D2, of greater than about 100.

The compounds of the invention interact with 5-HT receptors and act as antagonists at that receptor. The agonist or antagonist properties of the compounds were measured by the ability of the compounds to increase basal or to inhibit 5-HT-stimulated c-AMP formation in transfected cells expressing 5-HT₇ receptors. The biological activity of the inventive compounds is determined by assays that have been devised to serve as animal models for various human medical conditions. Many such assays are known to skilled practitioners. Examples of such assays include, e.g.:
- the prokinetic assay, which is an in vivo method of determining the extent the test compound affects the rate of gastric emptying of a test meal in rats;
- the anxiolytic behavior assay, which measures the extent to which the test compound can ameliorate of the symptoms of natural anxiety in mice when exposed to a novel, brightly lighted environment;
- the withdrawal anxiety assay, which measures the extent to which the test compound can ameliorate of the symptoms in mice caused by withdrawal from addictive substances by measuring the extent the drug affects the anxiety that occurs in mice after chronically treating with an addictive substance and then abruptly ceasing the treatments;
- the cognitive enhancement assay, which measures the extent the test compound can alleviate the cognitive deficit induced in rats by administration of atropine to rats.

These assays are described in U.S. Patent No. 5,763,468, the disclosure of which is hereby incorporated herein by reference.

The invention encompasses pharmaceutical compositions comprising compounds of Formula I, or a pharmaceutically acceptable salt or, solvate, thereof. As 5-HT₇ receptor ligands, the compounds of the invention are useful for treating conditions which can be ameliorated by interaction with 5-HT₇ receptors. Such conditions include sleep disorders, depression, pain, and schizophrenia.

"Aprodrug" is intended to mean a compound that is converted under physiological conditions or by solvolysis or metabolically to a specified compound that is pharmaceutically active. A "pharmaceutically active metabolite" is intended to mean a pharmacologically active compound produced through metabolism in the body of a specified compound. Prodrugs and active metabolites of compounds of Formulas I-V may be determined using techniques known in the art, for example, through metabolic studies. See, e.g., "Design of Prodrugs," (Bundgaard, ed.), 1985, Elsevier Publishers B.V., Amsterdam, The Netherlands.

A "pharmaceutically acceptable salt" is intended to mean a salt that retains the biological effectiveness of the free acids and bases of a specified compound and that is not biologically or otherwise undesirable. Examples of pharmaceutically acceptable salts include sulfates, pyrosulfates, bisulfates, sulfites, bisulfites, phosphates, monohydrogenphosphates, dihydrogenphosphates, metaphosphates, pyrophosphates, chlorides, bromides, iodides, acetates, propionates, decanoates, caprylates, acrylates, formates, isobutyrates, caproates, heptanoates, propiolates, oxalates, malonates, succinates, suberates, sebacates, fumarates, maleates, butyne-1,4-dioates, hexyne-1,6-dioates, benzoates, chlorobenzoates, methylbenzoates, dinitrobenzoates, hydroxybenzoates, methoxybenzoates, phthalates, sulfonates, xylenesulfonates, phenylacetates, phenylpropionates, phenylbutyrates, citrates, lactates, γ-hydroxybutyrates, glycollates, tartrates, methane-sulfonates, propanesulfonates, naphthalene-1-sulfonates, naphthalene-2-sulfonates, and mandelates. A "solvate" is intended to mean a pharmaceutically acceptable solvate form of a specified compound that retains the biological effectiveness of such compound. Examples of solvates include compounds of the invention in combination with water, isopropanol, ethanol, methanol, DMSO, ethyl acetate, acetic acid, or ethanolamine. In the case of compounds, salts, or solvates that are solids, it is understood by those skilled in the art that the inventive compounds, salts, and solvates may exist in different crystal forms, all of which are intended to be within the scope of the present invention and specified formulas.

Administration of the compounds of the invention and their pharmaceutically acceptable prodrugs, salts, active metabolites, and solvates may be performed according to any of the accepted modes of administration available to those skilled in the art. Illustrative examples of suitable modes of administration include oral, systemic (e.g., transdermal, intranasal, or by suppository), parenteral (e.g., intramuscular, intravenous, or subcutaneous), topical, transdermal and rectal. An inventive compound or a pharmaceutically acceptable salt, prodrug, active metabolite, or solvate thereof may be administered as a pharmaceutical composition in any pharmaceutical form recognizable to the skilled artisan as being suitable. Suitable pharmaceutical forms include solid, semisolid, liquid, or lyophilized formulations, such as tablets, powders, capsules, suppositories, suspensions, liposomes, and aerosols. Pharmaceutical compositions of the invention may also include suitable excipients, diluents, vehicles, and carriers, as well as other pharmaceutically active agents, depending upon the intended use or mode of administration. Acceptable methods of preparing suitable pharmaceutical forms of the pharmaceutical compositions are known or may be routinely determined by those skilled in the art. For example, pharmaceutical preparations may be prepared following conventional techniques of the pharmaceutical chemist involving steps such as mixing, granulating, and compressing when necessary for tablet forms, or mixing, filling, and dissolving the ingredients as appropriate, to give the desired products for oral, parenteral, topical, intravaginal, intranasal, intrabronchial, intraocular, intraaural, and/or rectal administration. Solid or liquid pharmaceutically acceptable carriers, diluents, vehicles, or excipients may be employed in the pharmaceutical compositions. Illustrative solid carriers include starch, lactose, calcium sulfate dihydrate, terra alba, sucrose, talc, gelatin, pectin, acacia, magnesium stearate, and stearic acid. Illustrative liquid carriers include syrup, peanut oil, olive oil, saline solution, and water. The carrier or diluent may include a suitable prolonged-release material, such as glyceryl monostearate or glyceryl distearate, alone or with a wax. When a liquid carrier is used, the preparation may be in the form of a syrup, elixir, emulsion, soft gelatin capsule, sterile injectable liquid (e.g., solution), or a nonaqueous or aqueous liquid suspension.

The compounds (active ingredients) may be formulated into solid oral dosage forms which may contain, but are not limited to, the following inactive ingredients: diluents (i.e., lactose, corn starch, microcrystalline cellulose), binders (i.e., povidone, hydroxypropyl methylcellulose), disintegrants (i.e., crospovidone, croscarmellose sodium), lubricants (i.e., magnesium stearate, stearic acid), and colorants (FD&C lakes or dyes). Alternatively, the compounds may be formulated into other oral dosage forms including liquids, suspensions, emulsions, or soft gelatin capsules, with each dosage form having a unique set of ingredients.

A dose of the pharmaceutical composition contains at least a therapeutically effective amount of the active compound or agent (i.e., an inventive compound or a pharmaceutically acceptable salt, prodrug, active metabolite, or solvate thereof), and preferably is made up of one or more pharmaceutical dosage units. The selected dose may be administered to a mammal, for example, a human patient, in need of treatment mediated by inhibition of serotonin agonist activity, by any known or suitable method of administering the dose, including topically, for example, as an ointment or cream; orally; rectally, for example, as a suppository; parenterally by injection; or continuously by intravaginal, intranasal, intrabronchial, intraaural, or intraocular infusion. A "therapeutically effective amount" is intended to mean the amount of an inventive compound that, when administered to a mammal in need thereof, is sufficient to effect treatment for disease conditions alleviated by the inhibition of the action of serotonin at the 5-HT receptor. The amount of a given compound of the invention that will be therapeutically effective will vary depending upon factors such as the particular compound, the disease condition and the severity thereof, the age and health of the subject in need of treatment, which may be routinely determined by skilled artisans.

The Examples that follow are intended as illustrations of certain preferred embodiments of the invention, and no limitation of the invention is implied. It is considered within the skill of one in the art to recognize that the chemical reactions described herein are generally applicable to prepare other compounds encompassed within the scope of the invention, or that such compounds may be prepared by appropriate modification of these illustrated reactions or use of analogous or other conventional synthetic methods known in the art, without undue experimentation (e.g., by use of appropriate blocking or protecting groups, by substituting other conventional reagents, or by routine modifications of reaction conditions). Although certain protecting groups are exemplified in the syntheses described below, it is understood that other suitable protecting groups may be used, depending on the functionality present in the desired compound and intermediates required for the preparation thereof, and depending on the particular synthesis method employed

In each of the synthetic procedures described herein, unless otherwise indicated, the starting materials are known, available, or may be readily prepared from known starting materials, all temperatures are set forth in degrees Celsius, and all parts and percentages are by weight. Reagents were purchased from commercial suppliers, such as Aldrich Chemical Company or Lancaster Synthesis Ltd. Reagents and solvents were commercial grades and were used as supplied. ¹H-NMR (300 MHz) spectra were measured in CDCl₃ solutions unless otherwise indicated and were determined on a Bruker DRX-300 instrument using XWIN NMR Version 1.2 operating software. Chemical shifts are reported in parts per million (ppm) downfield from tetramethylsilane as the internal standard, and coupling constants are given in Hertz. The following abbreviations are used for spin multiplicity: br = broad, s = singlet, d = doublet, t = triplet, q = quartet, m = multiplet, and cm = complex multiplet. Infrared (IR) spectra were recorded on a Perkin-Elmer 1600 series FTIR spectrometer and are reported in wavenumbers (cm⁻¹). Elemental analyses were performed by Atlantic Microlab, Inc., Norcross, GA. High-resolution mass spectra (HRMS) were performed by Scripps Mass Spectra Laboratory, La Jolla, CA. Melting points (mp) were determined on a Mel-Temp II apparatus and are uncorrected. Unless otherwise indicated, the reactions set forth below were carried out under a positive pressure with a balloon of nitrogen (N₂) or argon (Ar) at ambient temperature in anhydrous solvents, and the reaction flasks were fitted with rubber septa for the introduction of substrates and reagents via syringe. Glassware was heat-dried. Analytical thin-layer chromatography (TLC) was performed on glass-backed silica gel 60 F 254 plates (Analtech, 0.25 mm) and eluted with the appropriate solvent ratios (v/v), which are denoted where appropriate. The reactions were assayed by TLC and terminated as judged by the consumption of starting material. The tip plates were visualized using an ultraviolet (UV) lamp. Visualization can also be accomplished using stains such as potassium permanganate, ninhydrin, ammonium molybdate, iodine (I₂) chamber, or *p*-anisaldehyde spray reagent or phosphomolybdic acid reagent (Aldrich Chemical, 20 wt% in ethanol) activated with heat.

Recovery of the desired compounds from the reaction mixtures described herein was typically accomplished by doubling the reaction volume with the reaction solvent or extraction solvent and washing with the indicated aqueous solutions using 25% by volume of the extraction volume (unless otherwise indicated). Product solutions were dried over anhydrous Na₂SO₄ prior to filtration and evaporation of the solvents was conducted under reduced pressure on a rotary evaporator. Purification of products and intermediates was conducted by flash column chromatography using silica gel 60 (Merck Art 9385). (Still et al., *J. Org. Chem.* 43:2923 (1978)) was done using silica gel 60 (Merck Art 9385):crude material ratio of about 20:1 to 50:1 (unless otherwise indicated).

### Intermediate 1

### (S)-2-Aminoethyl-1-methylpyrrolidine was prepared according to the following reaction scheme:

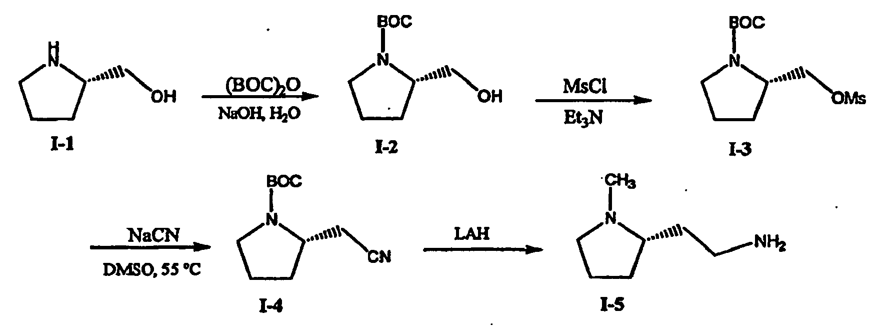

(S)-N-Boc-2-hydroxymethylpyrrolidine (1-2):, t-Butoxycarboxylic anhydride (23.3 g,107 mmol) was added to a solution of 5.40 g of (S)-2-hydroxymethylpyrmlidine (I-1, 53.4 mmol) in 100 mL of 1.5 N aqueous sodium hydroxide (NaOH). After stirring for 1 hour, the reaction was extracted twice with ethylacetate (EtOAc). The EtOAc phases were combined, dried with magnesium sulfate (MgSO₄), filtered and concentrated under reduced pressure. The residue was purified on a silica gel column using hexane/EtOAc (3:1), followed by (1:1) to provide 10.72 g of the title product. ¹H NMR(CDCl₃, ppm): 1.48 (s, 9H); 1.53 (m, 1H); 1.7-1.9 (m, 4H); 2.0 (m, 1H; 3.30 (m, 1H); 3.45 (m, 1H); 3.60 (m, 2H); 3.95 (brd, 1H); 4.80 (brd, 1H). MS: 202 (M⁺+1).

(S)-Mesylate of N-Boc-2-hydroxymethylpyrrolidine (I-3): Mesyl chloride (4.8 mL, 61.5 mmol) was added slowly with stirring, at 0°C, to a solution of 10.3 g of N-Boc-2-hydroxymethylpyrrolidine (51.2 mmol) and triethylamine (7.76 g, 76.8 mmol) in 150 mL tetrahydrofuran (THF). After 40 minutes, the reaction mixture was filtered to remove solid formed, and the solvent was concentrated under reduced pressure. The residue was purified on a silica gel, column, using hexane/EtOAc (1:1), to provide 14.25 g (99.7%) of the title product. ¹H NMR (CDCl₃, ppm): 1.50 (s, 9H); 1.8 - 2.2 (m, 5H); 3.03 (s, 3H); 3.37 (brd, 2H); 4.05 (brd, 1H); 4.30 (brd, 1H).

(S)-N-Boc-2-cyanomethylpyrrolidine (I-4): The mesylate salt of N-Boc-2-hydroxymethylpyrrolidine (12.0 g, 43.0 mmol) and NaCN (6.32 g) were mixed in 50 mL of DMSO and stirred at 55°C for 20 hours. After cooling to room temperature, 200 mL of EtOAc was added and the mixture was washed successively with 10% aq. potassium carbonate (K₂CO₃, 1x) and brine (2x),.dried with MgSO₄ and filtered. The resulting solution was concentrated under reduced pressure. The residue was purified on a silica gel using hexane/EtOAc (1:1) to provide 8.2 g (91.1%) of the title product. ¹H NMR (CDCl₃, ppm): 1.43 (s, 9H); 1.75-2.05 (m, 3H); 2.13 (brd, 1H); 2.45 - 2.90 (m, 2H); 3.40 (m, 2H); 3.95 (brd, 1H), MS: 211 (M⁺+1).

(S)-2-Aminoethyl-1-methylpyrrolidine (I-5): Lithium aluminum hydride (201 mL of 1.0 M solution in THF) was added slowly to a solution of N-Boc-2-cyanomethylpyrrolidine (14.1 g, 31.0 mmol) in 500 mL of diethyl ether. After stirring at room temperature for 10 min., the reaction mixture was heated to reflux for 18 hours, cooled in ice bath, and quenched with 25% NaOH aqueous solution. The resulting mixture was filtered, and the solid was washed thoroughly with ether. The filtrate and combined washings were dried with sodium sulfate (Na₂SO₄), filtered, and concentrated under reduced pressure to provide 6.63 g (77.2%) of the title product. ¹H NMR (CDCl₃, ppm): 1.40 (s, 2H); 1.50 - 2.15 (m, 8H); 2.28 (s, 3H); 2.70 (m, 2H); 3.00 (m, 1H). MS:129 (M⁺+1).

The (R)-enantiomer of intermediate I-5 was made by the same procedure using the (R)-enantiomer of I-1 as starting material. Racemic intermediate I-5 is commercially available.

### EXAMPLE 1

(S)-Di-(3-hydroxybenzyl)-2-aminoethyl-N-methylpyrrolidine was prepared according to the following reaction scheme:

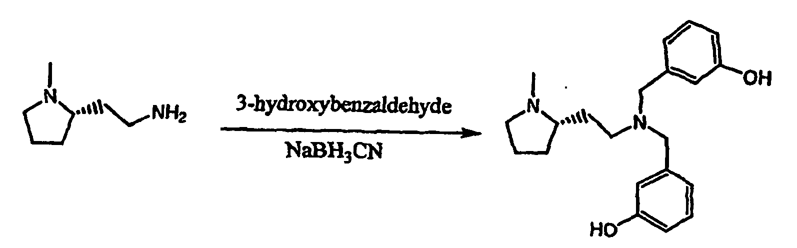

Sodium cyanoborohydride (5.0 g, 79.6 mmol), admixed with a few droplets of trifluoroacetic acid (TFA), was slowly added to a solution of (S)-2-aminoethyl-1-methylpyrrolidine (1.28 g, 10 mmol) and 3-hydroxybenzaldehyde (4:9 g, 40 mmol) in 30 mL of methanol. The resulting mixture was stirred for 48 hours, quenched with 2N HCl, and concentrated under reduced pressure. The residue was dissolved in MeOH, filtered and the resulting clear solution concentrated under reduced pressure. The residue was purified on a silica gel column using MeOH/CH₂Cl₂ (5:95 first, then changed to 15:85) to provide 3.04 g of the title compound. The compound was further purified using reverse phase HPLC using a C-18 column. ¹H NMR (CD₃OD): δ 1.34-2.21 (m, 7H), δ 2.62-2.82 (m, 2H), δ 2.84 (s, 3H), δ 3.10-3.27 (m, 1H), δ 3.48-3.85 (m, 4H), δ 4.12-4.32 (m, 1H), δ 6.70-7.06 (m, 6H), δ 7.15-7.45 (m, 2H). MS (APCI): 341.3 (M+1).

The compounds of Examples 3 to 12 were prepared according to the general procedure of Example 2, using the specified aldehydes.

### EXAMPLE 2

The following compound was prepared from racemic 2-aminoethyl-1-methylpyrrolidine and 3-hydroxybenzaldehyde: 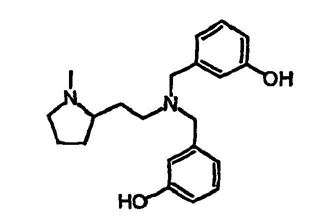
¹H NMR (DMSO-d₆): δ 1.97 (brd, 2H), δ 2.74 (d, 3H), δ 2.98 (brd, 3H), δ 3.52 (brd, 6H), δ 4.22 (brd, 4H), δ 6.87 (d, 2H), δ 7.06 (m, 4H), δ 7.25 (t, 2H), δ 9.75 (brd, 1H), δ 10.89 (brd, 1H). MS (APCI): 341.3 (M+1).

### EXAMPLE 3

The following compound was prepared from racemic 2-aminoethyl-1-methylpyrrolidine and p-tolualdehyde: 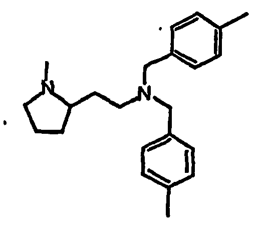
¹H NMR (CDCl₃): δ 1.21-1.44 (m, 2H), δ 1.52-1.76 (m, 3H), δ 1.95-2.15 (m, 3H), δ 2.29 (s, 3H), δ 2.35 (s, 6H), δ 2.30-2.60 (m, 2H), δ 3.05 (t, 1H), δ 3.44 (d, 2H), δ 3.65 (d, 2H), δ 7.08 (m, 4H), δ 7.23 (m, 4H). MS (APCI): 337.2 (M+1).

### EXAMPLE 4

The following compound was prepared from racemic 2-aminoethyl-1-methylpyrrolidine and 4-chlorobenzaldehyde: 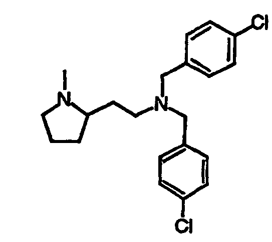
¹H NMR (CDCl₃): δ 1.24-1.60 (m, 2H), δ 1.64-1.87 (m, 3H), δ 1.92-2.06 (m, 1H), δ 2.14-2.32 (m, 2H), δ 2.35 (s, 3H), δ 2.43 (t, 2H), δ 3.19 (t, 1H), δ 3.37 (d, 2H), δ 3.60 (d, 2H), δ 7.26 (m, 8H). MS (APCI): 377 (M+1).

### EXAMPLE 5

The following compound was prepared from (R)-2-aminoethyl-1-methylpyrrolidine and p-tolualdehyde: 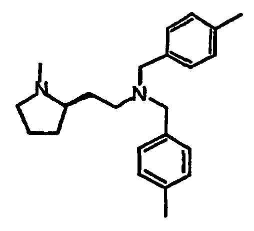
¹HNMR (CDCl₃): 81.21-1.48 (m, 2H), δ 1.54-1.83 (m, 2H), δ 1.87-2.24 (m, 4H), 82.26 (s, 3H), δ 2.32 (s, 6H), δ 2.36-2.53 (m, 2H), δ 3.05 (t, 1H), δ 3.43 (d, 2H), δ 3.60 (d, 2H), δ 7.10 (m, 4H), δ 7.22 (m, 4H). MS (GC): 337 (M+1).

### EXAMPLE 6

The following compound was prepared from (S)-2-aminoethyl-1-, methylpyrrolidine and p-tolualdehyde: 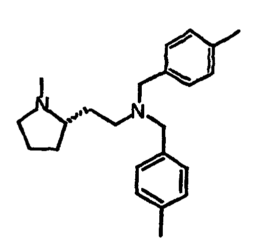
¹H NMR (CDCl₃): δ 1.21-1.48 (m, 2H), δ 1.54-1.83 (m, 2H), δ 1.87-2.24 (m, 4H), δ 2.26 (s, 3H), δ 2.32 (s, 6H), δ 2.36-2.53 (m, 2H), δ 3.05 (t, 1H), δ 3.43 (d, 2H), δ 3.60 (d, 2H), δ 7.10 (m, 4H), δ 7.22 (m, 4H). MS (GC): 337 (M+1).

### EXAMPLE 7

The following compound was prepared from racemic 2-aminoethyl-1-methylpyrrolidine and phenylacetaldehyde: 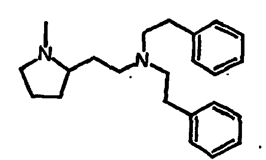
¹H NMR (CDCl₃): δ 1.31-1.58 (m, 2H), δ 1.60-1.87 (m, 2H), δ 1.87-2.24 (m, 4H), δ 2.29 (s, 3H), δ 2.60 (t, 2H), δ 2.68-2.91 (m, 6H), δ 3.06 (t, 1H), δ 7.08-7.42 (m, 10H). MS (APCI): 337.8 (M+1).

### EXAMPLE 8

The following compound was prepared from (S) 2-aminoethyl-1-methylpyrrolidine and 3-(chloromethyl)benzamide): 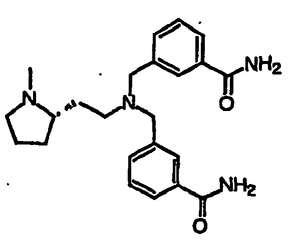
(S)-2-Aminomethyl-1-methylpyrrolidine (0.20 g, 1.6 mmol) and 3-(chloromethyl)benzamide (0.75 g, 4.4 mmol) were mixed in 15 mL MeOH and stirred overnight. The reaction mixture was subjected to HPLC to provide purified compound 9. ¹H NMR (CD₃OD): δ1.62 (m, 1H), δ1.95-2.30 (m, 4H), δ 2.63 (m, 1H), δ 2.91 (s, 3H). δ 3.08-3.45 (m, 4H), δ 3.60-3.81 (m, 1H), δ 4.40-4.66 (m, 4H), δ 7.41-8.10 (m, 8H). MS (LC-MS): 395.5 (M+1).

### EXAMPLE 9

The following compound was prepared from (R)-2-aminoethyl-1-methylpyrrolidine and 3-hydroxybenzaldehyde: 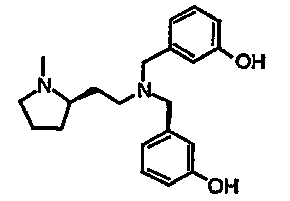
¹H NMR (DMSO-d₆): δ1.10-2.30 (m, 6H), δ 2.61 (s, 3H), δ 2.76-3.50 (m, 5H), δ 3.94-4.29 (m, 4H), δ 6.70-7.30 (m, 8H), δ 11.10 (d, 2H). MS (APCI): 341.12 (M+1).

### EXAMPLE 10

The following compound was prepared from (S)-2-aminoethyl-1-methylpyrrolidine and 3,5-dimethyl-4-hydroxybenzaldehyde: 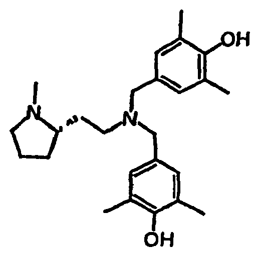
¹H NMR (CDCl₃): δ 1.43-1.62 (m, 1H), δ 1.87-2.12 (m, 4H), δ 2.15 (s, 12H), δ 2.25-2.40 (m, 1H), δ 2.82 (s, 3H), δ 2.95-3.20 (m, 4H), δ 3.55-3.66 (m, 1H), δ 4.02-4.26 (m, 4H), δ 6.93-7.07 (m, 4H). MS (APCI): 397.3 (M+1).

### EXAMPLE 11

The following compound was prepared from racemic 2-aminoethyl-1-methylpyrrolidine and benzaldehyde: 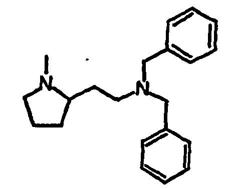
¹H NMR (CDCl₃): δ 1.19-1.44 (m, 2H), δ 1.55-1.79 (m, 2H), δ 1.86-2.17 (m, 4H), δ 2.26 (s, 3H), δ 2.43-2.55 (m, 2H), δ 3.05 (m, 1H), δ 3.44 (d, 2H), δ 3.66 (d, 2H), δ 7.16-7.47 (m, 10H). MS (GC-EI): 309 (M+1).

### Intermediate 2

(S)-(3-Hydroxybenzyl)-2-aminoethyl-N-methylpyrrolidine was prepared according to the following:

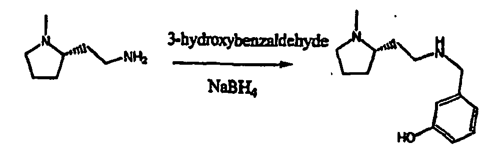

(S)-2-Aminoethyl-1-methylpyrrolidine (1.28 g, 10 mmol) and 3-hydroxybenzaldehyde (1.22 g, 10 mmol) were dissolved in 25 mL of MeOH, and the resulting solution was stirred overnight. After the reaction mixture was cooled in an ice bath, sodium borohydride (NaBH₄, 0.76 g) was added slowly in aliquots. The reaction mixture was stirred for 15 minutes while chilled, and for another 1.5 hours at room temperature. The reaction mixture was cooled down in an ice bath, quenched slowly with TFA, and the solvent was concentrated under reduced pressure. The residue was purified on a silica gel column using EtOAc/MeOH (2:1). The product obtained was converted to the hydrochloride salt, providing 2.7 g (88%) . ¹H NMR (2HCl salt in DMSO, ppm): 1.60 (m, 1H); 1.90 (m, 2H); 2.10 (m, 1H); 2.25 (m, 1H); 2.40 (m, 1H); 2.74 (d, 3 H); 3.00 (m, 3H); 3.38 (m, 1H); 3.50 (m, 1H0; 4.00 (m, 2H); 6.83 (m, 1H); 7.00 (m, 2H); 7.19 (t, 1H); 9.70 (brd, 2H); 11.2 (bred, 1H). MS (APCI): 235 (M⁺+1).

The intermediate compounds of Examples 14 to 18 were prepared according to the general procedure of Example 13 using the specified aldehydes.

### Intermediate 3

The following intermediate compound was prepared from 2-aminoethyl-1-methylpyrrolidine and benzaldehyde: 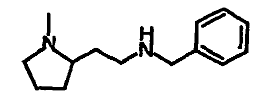
¹H NMR (CDCl₃): δ 1.44-1-52 (m, 2H), δ 1.53-1.80 (m, 3H), δ 1.85-1.95 (m, 2H), δ 2.04-2.15 (m, 2H), δ 2.30 (s, 3H), δ 2.61-2.64 (m, 1H), δ 2.64-2.74 (m, 1H), δ 3.05 (t, 1H), δ 3.80 (m, 2H), δ 726 (d, 1H), δ 7.32 (m, 4H). MS (APCI): 219.2 (M+1).

### Intermediate 4

The following intermediate compound was prepared from 2-aminoethyl-1-methylpyrrolidine and 2-naphthaldehyde: 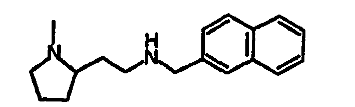
¹H NMR (CDCl₃): δ 1.38-1.61 (m, 2H), δ 1.63-1.83 (m, 2H), δ 1.84-2.03 (m, 2H), δ 2.05-2.23 (m, 2H), δ 2.32 (s, 3H), δ 2.60-2.84 (m, 2H), δ 3.05 (t, 1H), δ 3.95 (s, 2H), δ 7.36 - 7.60 (m, 3H), δ 7.95 (m, 4H). MS (FAB): 269.4 (M+1).

### Intermediate 5

The following intermediate compound was prepared from 2-aminoethyl-1-methylpyrrolidine and 4-chlorobenzaldehyde: 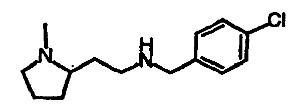
¹H NMR (CDCl₃): δ 1.36-1.61 (m, 2H), δ 1.62 - 2.00 (m, 4H), δ 2.01-2.20 (m, 2H), δ 2.29 (s, 3H), δ 2.53-2.79 (m, 2H), δ 3.08 (t, 1H), δ 3.75 (s, 2H), δ 7.17-7.41 (M, 4H). MS (FAB): 253.2 (M+1).

### Intermediate 6

The following intermediate compound was prepared from 2-aminoethyl-1-methylpyrrolidine and p-tolualdehyde: 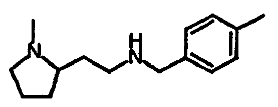
¹H NMR (CDCl₃): δ 1.35-1.52 (m, 2H), δ 1.56-1.80 (m, 2H), δ 1.84-1.99 (m, 4H), δ 2.00-2.16 (m, 2H), δ 2.30 (s, 3H), δ 2.34 (s, 3H), δ 2.56 - 2.79 (m, 2H), δ 3.04 (t, 1H), δ 3.75 (s, 2H), δ 7.07-7.24 (dd, 4H). MS (GC): 232 (M).

### Intermediate 7

The following intermediate compound was prepared from 2-aminoethyl-1-methylpyrrolidine and 2-quinolinecarboxaldehyde: 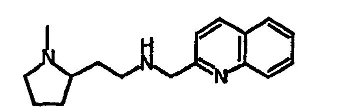
¹H NMR (CDCl₃): δ 1.42-1.87 (m, 4H), δ 1.88-2.05 (m, 2H), δ 2.06-2.24 (m, 2H), δ 2.34 (s, 3H), δ 2.66-2.89 (s, 2H), δ 3.07 (t, 1H), δ 4.11 (s, 2H), δ 7.42 - 7.56 (m, 2H), δ 7.67 (t, 1H), δ 7.80 (d, 1H), δ 8.02-8.21 (m, 2H). MS (APCI): 270.3 (M+1).

### EXAMPLE 12

(S)-(3-Hydroxybenzyl)(4-chlorobenzyl)-2-aminoethyl-N-methylpyrrolidine was prepared as follows:

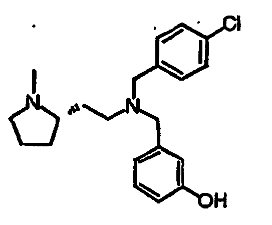

To a stirred solution of S-2-(3-hydroxybenzyl)aminoethyl-1-methylpyrrolidine (0.31 g, 1.0 mmol) and p-chlorobenzaldehyde (0.28 g, 2.0 mmol) in 8 mL of MeOH, was added a sodium cyanoborohydride (0.28 g, 4.46 mmol), followed by a droplet of TFA. The resulting mixture was stirred for 24 hours at 55°C, followed by addition of 2N HCl. The mixture was concentrated under reduced pressure and the resulting residue was dissolved in aqueous HCl. The aqueous solution was extracted with EtOAc and concentrated under reduced pressure. The residue was subjected to purification by reverse phase HPLC (C₁₈, reverse phase column) to provide the title compound (106 mg, 30%). ¹H NMR (CD₃OD): δ 1.51 - 1.73 (m, 1H), δ 1.92 - 2.29 (m, 4H), δ 236 - 2.58 (m, 1H), δ 2.92 (s, 3H), δ 3.06-3.29 (m, 4H), δ 3.66-3.83 (m, 1H), δ 4.27-4.55 (m, 4H), δ 6.86-7.04 (m, 3H), δ 7.27-7.40 (m, 1H), δ 7.50-7.64 (M, 4H). MS (APCI):359.8 (M+1).

The compounds of Examples 20 to 53 were prepared according to the general procedure of Example 19, or by straightforward modification thereof, using the intermediate 2-aminoethyl-N-methylpyrmlidines of Examples 13 to 18, or related pyrrolidine intermediates prepared by straightforward modification of the general procedure of Example 13, and commercially available aldehydes.

### EXAMPLE 13

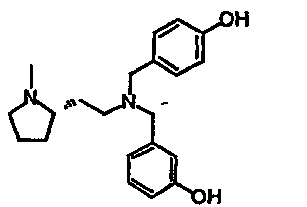

¹H NMR (CD₃OD): δ 1.46 -1.72 (m, 1H), δ 1.90-2.29 (m, 4H), δ 2.31 - 2.50 (m, 1H), δ 2.87 (s, 3H), δ 3.01 - 3.31 (m, 4H), δ 3.60 - 3.78 (m, 1H), δ 4.30 (s, 4H), δ 6.76 - 7.03 (m, 5H), δ 7.22 - 7.44 (m, 3H). MS (APCI): 341.4 (M+1).

### EXAMPLE 14

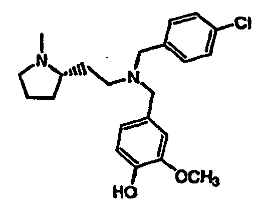

¹H NMR (CD₃OD): δ1.54 (m, 1H), δ 1.90 - 2.22 (m, 4H), δ 2.39 (m, 1H), δ2.83 (s, 1H), δ 2.97 - 3.39 (m, 4H), δ 3.59 (m, 1H), δ 3.82 (s, 3M, δ 4.05 - 4.64 (m, 4H) δ 6.73 - 7.01 (m, 2H), δ 7.13 (s, 111), δ 7.35 - 7.64 (m, 4H). MS (APCI): 389.2 (M+1).

### EXAMPLE 15

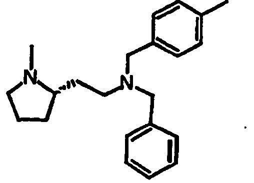

¹H NMR (CDCl₃): δ 0.76 -1.00 (m, 1H), δ 1.18 - -1.47 (m, 3H), δ 1.55 - 2.11 (m, 4H), δ 2.25 - 2.89 (m, 9H), δ 3.30 - 3.56 (m, 2H), δ 3.59 - 3.77 (m, 1H), δ 4.60 (m, 1H), δ 7.00 - 7.49 (m, 9H). MS (APCI): 323.1 (M+1).

### EXAMPLE 16

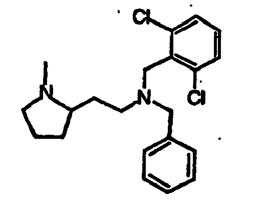

¹H NMR (CDCl₃): δ 0.74 - 1.42 (m, 4H), δ 1.45 - -1.76 (m, 2H), δ 1.80 - 2.14 (m, 2H), δ 2.14 - 3.83. (m, 5H), δ 3.33 - 4.02 (m, 4H), δ 4.95 (m, 1H), δ 7.08 - 7.40 (m, 8H). MS (APCI): 378 (M+1).

### EXAMPLE 17

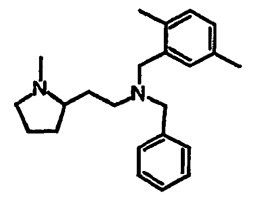

¹H NMR (CDCl₃): δ 0.94 -1.72 (m, 6H), δ 1.74 - 2.05 (m, 2H), δ 2.13 - 2.57 (m, 14H), δ 3.56 - 3.70 (m, 2H), δ 3.33 - 3.47 (m, 2H), δ5.21 - 5.63 (m, 1H), δ 6.89 - 7.41 (m, 8H). MS (APCI): 337.1 (M+1).

### EXAMPLE 18

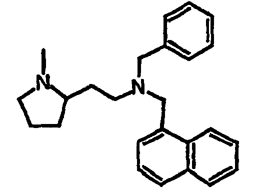

¹H NMR (CDCl₃): δ 0.99 - 2.05 (m, 6H), δ 2.11 - 2.63 (m, 7H), 63.21 - 4.24 (m, 5H), δ 7.10 - 8.19'(m, 12H). MS (APCI): 359.1 (M+1).

### EXAMPLE 19

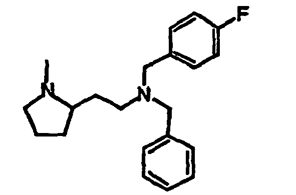

¹H NMR (CDCl₃): δ 0.92 - 2.08 (m, 6H), δ 2.27 - 2.54 (m, 7H), δ 331 - 3.48 (m, 2H), δ 3.57 - 3.71 (m, 2H), δ 6.99 (t, 2H), δ 7.29 (m, 7H). MS (APC1): 327.1 (M+1).

### EXAMPLE 20

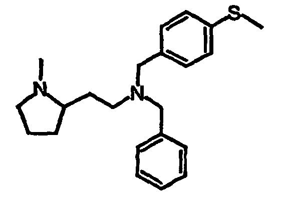

¹H NMR (CDCl₃): δ 0.98 -1.73 (m, 4H), δ 1.85 - 2.11 (m, 2H), δ 2.19 - 3.02 (m, 10H), δ 3.14 - 4.29 (m, 5H), δ.04 - 7.41 (m, 9H). MS (APCI): 355.1 (M+1).

### EXAMPLE 21

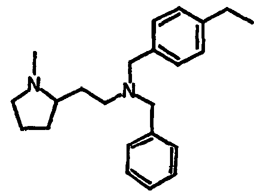

¹H NMR (CDCl₃): δ 0.95 - 1.43 (m, 4H), δ 1.44 - 2.10 (m, 6H), δ 2.21 - 2.70 (m, 8H), δ 3.21 - 3.36 (m, 1H), δ 3.37 - 3.50 (m, 2H), δ 3.57 - 3.72 (m, 2H), δ 7.04 - 7.50 (m, 9H). MS (APCI): 337.1 (M+1).

### EXAMPLE 22

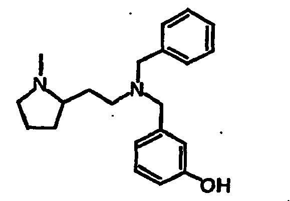

¹H NMR (CDCl₃): δ 0.92 - -1.98 (m, 6H), δ 2.21 - 3.10 (m, 7H), δ 3.30 - 4.13 (m, 5H), δ 6.68 - 7.45 (m, 9H). MS (APCI): 325.2 (M+1).

### EXAMPLE 23

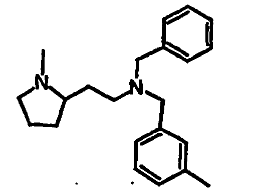

¹H NMR (CDCl₃): δ 0.89 - 2.12 (m, 9H), δ 2.22 - 2.56 (m, 7H), δ 3.27 (t, 1H), δ 3.40 - 3.56 (m, 2H), δ 3.81 - 3.60 (m, 2H), δ 6.96 - 7.45 (m, 9H). MS (APCI): 323.2 (M+1).

### EXAMPLE 24

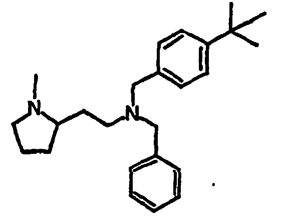

¹H NMR (DMSO-d₆): δ 1.16 (m, 1H), δ 1.64 (m, 2H), δ 1.95 (m, 1H), δ 2.26 (s, 5H), δ 2.48 (d, 2H), δ 2.75 (m, 2H), δ 3.25 (m, 1H), δ 3.67 (s, 9H), δ 3.96 - 4.32 (m, 4H), δ 6.67 - 7.52 (m, 9H). MS (APCI): 365.2 (M+1).

### EXAMPLE 25

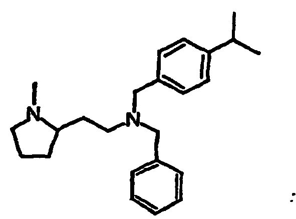

¹H NMR (DMSO-d₆): δ 0.80 - 2.13 (m, 8H), δ 2.40 (s, 3H), δ 2.58 (m, 2H), δ 2.61 (s, 6H), δ 2.90 (m, 1H), δ 3.36 - 3.81 (m, 4H), δ 4.67 (s, 1H), δ 7.17 - 8.49 (m, 9H). MS (APCI): 351.2 (M+1).

### EXAMPLE 26

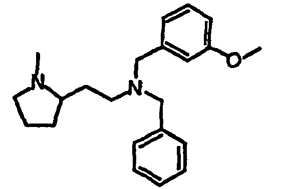

¹H NMR (CDCl₃): δ 0.74 - 2.33 (m, 8H), δ 2.41 (s, 3H), δ 2.48 - 2.78 (m, 2H), δ 3.34 - 3.70 (m, 4H), δ 3.60 - 3.80 (m, 1H), δ 3.82 - 4.00 (s, 3H), δ 6.70 - 7.50 (m, 9H). MS (APCI):339.1(M+1).

### EXAMPLE 27

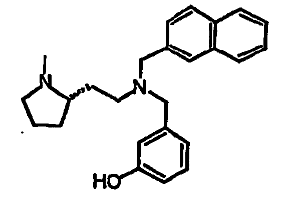

¹H NMR (DMSO-d₆): δ1.77 - 2.42 (m, 6H), δ 2.72 (s, 3H), δ 2.86 - 3.18 (m, 4H), δ 3.23 (m, 1H), δ 4.15 - 4.70 (m, 4H), δ 6.81 - 8.35 (m, 11H), δ 9.86 (s, 1H). MS (APCI): 375.5 (M+1).

### EXAMPLE 28

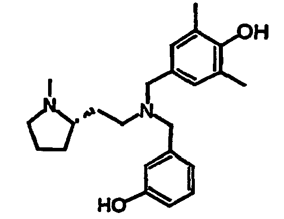

¹H NMR (DMSO-d₆): δ 1.39 - 1.58 (m, 1H), δ 1.80 - 2.05 (m, 4H), δ 2.06 - 2.32 (m, 7H), δ 2.75 (s, 3H), δ 2.84 - 3.10 (m, 4H), δ 3.16 - 3.35 (m, 1H), δ 4.00 - 4.35 (m, 4H), δ 6.81 - 6.92 (m, 1H), δ 7.01 - 7.14 (m, 2H), δ 7.18 - 7.30 (m, 1H), δ 7.36 (s, 1H), δ 7.55 (s, 1H). MS (APCI): 369.8 (M+1).

### EXAMPLE 29

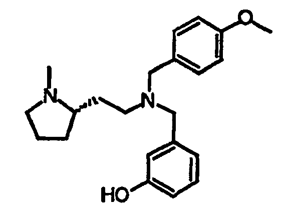

¹H NMR (DMSO-d₆): δ 1.34-2.60 (m,6H), δ 2.77 (s, 3H), δ 2.84-3.10 (m,4H), δ 3.15 (m, 1H), δ 3.80 (s, 3H), δ 4.08 - 4.40 (m, 4H), δ 6.82 - 6.93 (m, 1H), δ 6.93 - 7.12 (m, 4H). δ 7.18 - 7.32 (m, 2H), δ 7.39 (s, 1H), δ 7.55 (s, 1H), δ 7.61 - 7.74 (m, 2H). MS (LC-MS): 355.4 (M+1).

### EXAMPLE 30

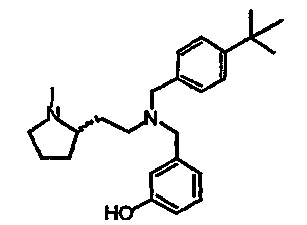

¹H NMR (DMSO-d₆): δ 1.34 - 1.55 (m, 10H), δ 1.77 - 2.15 (m, 4H), δ 2.30 - 2.52 (m, 1H), δ 2.77 (s, 3H), δ 2.93 - 3.31 (m, 4H), δ 3.63 (m, 1H), δ 4.08 - 4.53 (m, 4H), δ 6.79 - 7.02 (m, 3H), δ 7.20 - 7.35 (m, 1H), δ 7.41 - 7.61 (m, 4H), δ 10.36 (s, 1H). MS (APCI): 381.6 (M+1).

### EXAMPLE 31

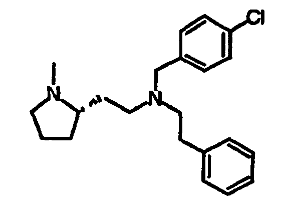

¹H NMR (CD₃OD): δ 1.60 - -1.83 (m, 1H), δ 1.94 - 2.01 (m, 3H), δ 2.03 - 2.44 (m, 1H), δ 2.45 - 2.65 (m, 1H), δ 2.95 (s, 3H), δ 3.04 - 3.23 (m, 2H), δ 3.25 - 3.53 (m, 6H), δ 3.65 - 3.84 (m, 1H), δ 4.40 - 4.67 (m, 2H), δ 7.24 - 7.43 (m, 3H), δ 7.51 - 7.72 (m, 4H). MS (APCI): 357.8 (M+1).

### EXAMPLE 32

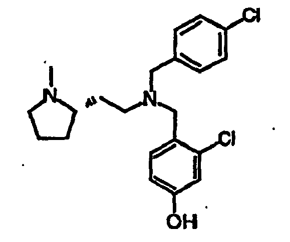

¹H NMR (CD₃OD): δ 1.58 - 1.81 (m, 1H), δ 1.92 - 2.36 (m, 4H), δ 2.41 - 2.68 (m, 1H), δ 2.92 (s, 3H), δ 3.07 - 3.52 (m, 6H), δ 3.61 - 3.84 (m, 1H), δ 4.28 - 4.50 (m, 4H), δ 6.72 - 7.04 (m, 2H), δ 7.34 - 7.66 (m, 5H). MS (APCI): 393.9 (M+1).

### EXAMPLE 33

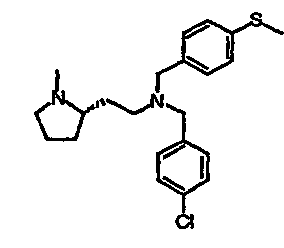

¹H NMR (CD₃OD): δ 1.53 -1.77 (m, 1H), δ 1.94 - 2.31 (m, 4H), δ 2.39 - 2.62 (m, 4H), δ 2.94 (s, 3H), δ 3.06 - 3.31 (m, 4H), δ 3.66 - 3.84 (m, 1H), δ 4.32 - 4.55 (m, 4H), δ 7.31- 7.64 (m, 8H). MS (APCI): 389.6 (M+1).

### EXAMPLE 34

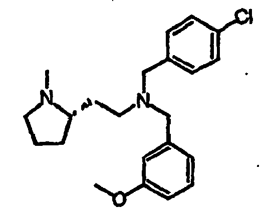

¹H NMR (CD₃OD): δ 1.29 - 1.53 (m, 1H), δ 1.71 - 2.08 (m, 4H), δ 2.15 - 2.38 (m, 1H), δ 2.68 (s, 3H), δ 2.87 - 3.16 (m, 4H), δ 3.41 - 3.61 (m, 4H), δ 4.07 - 4.36 (m, 4H), δ 6.79 - 7.00 (m, 3H), δ 7.14 - 7.44 (m, 5H). MS (APCI): 373.4 (M+1).

### EXAMPLE 35

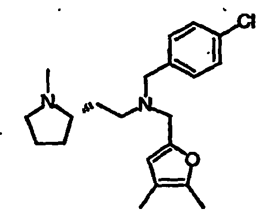

¹H NMR (CDCl₃): δ 1.73 - -1.90 (m, 1H), δ 1.96 (s, 3H), δ 1.98 - 2.09 (m, 2H), δ 2.11 - 2.23 (m, 1H), δ 2.26 (s, 3H), δ 2.35 - 2.62 (m, 2H), δ 2.76 - 2.94 (m, 4H), δ 2.95 - 3.10 (m, 1H), δ 3.11 - 3.24 (m, 1H), δ 3.25 - 3.43 (m, 1H), δ 3.77 - 3.94 (m. 1H). δ 4.04 - 4.32 (m, 4H), δ 6.46 (s, 1H), δ 7.40 - 7.70 (m, 4H). MS (APCI): 361.2 (M+1).

### EXAMPLE 36

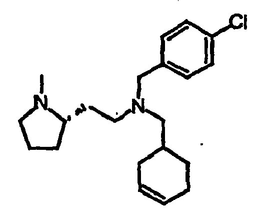

¹H NMR (CDCl₃): δ 1.15 - 1.21 (m, 1H), δ 1.57 - 1.94 (m, 3H), δ 1.95 - 2.32 (m, 7H), δ 2.36 - 2.63 (m, 2H), δ 2.78 - 2.92 (m, 6H), δ 3.03 - 3.25 (m, 1H), δ 3.26 - 3.50 (m, 2H), δ 3.64 - 3.83 (m, 1H), δ 4.09 - 4.45 (m, 2H), δ 5.53 - 5.80 (m, 2H), δ 7.33 - 7.64 (m, 4H). MS (APCI): 347.5 (M+1).

### EXAMPLE 37

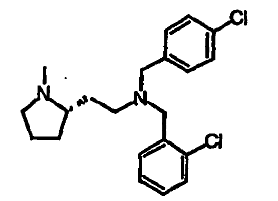

¹H NMR (CD₃OD): δ 1.63 1.92 (m, 1H), δ 2.00 - 2.44 (m, 4H), δ 2.57 - 2.79 (m, 1H), δ 2.99 (s, 3H), δ 3.10 - 3.29 (m, 1H), δ 3.31 - 3.57 (m, 3H), δ 3.65 - 3.84 (m, 1H), δ 4.40 - 4.82 (m, 4H), δ 7.36 - 7.92 (m, 8H). MS (APCI): 377.6 (M+1).

### EXAMPLE 38

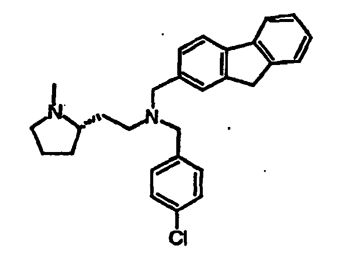

¹H NMR (CD₃OD): δ 1.54 -1.78 (m, 4H), δ 2.47 - 2.74 (m, 1H), δ 2.95 (s, 3H), δ 3.09 - 3.52 (m, 4H), δ 3.60 - 3.82 (m, 1H), δ 3:96 (s, 2H), δ 4.42 - 4.73 (m, 4H), δ 7.26 - 8.00 (m, 11H). MS (APCI): 431.5 (M+1).

### EXAMPLE 39

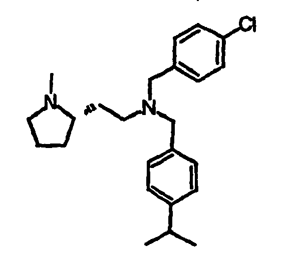

¹H NMR (CD₃OD). δ 1.26 (d, 6H), δ 1.50 - 1.71 (m, 1H), δ1.92 - 2.28 (m, 4H), δ 2.37- 2.59 (m, 1H), δ 2.90 (s, 3H), δ 3.05 - 3.35 (m, 4H), 6 3.61 - 3.80 (m, 1H), δ 4.29 - 4.54 (m, 4H), δ 7.29 - 7.58 (m, 8H). MS (APCI): 385.6 (M+1).

### EXAMPLE 40

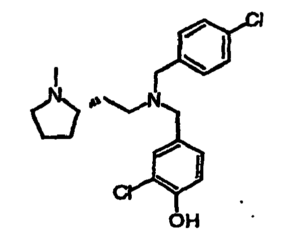

¹H NMR (CD₃OD): δ 1.46 - 1.64 (m, 1H), δ 1.85 - 2.19 (m, 4H), δ 2.34 - 2.52 (m, 1H), δ 2.83 (s, 3H), δ 2.96 - 3.18(m, 3H), δ 3.19 - 3.26 (m, 1H), δ 3.52 - 3.64 (m 1H), δ 4.17 - 4.45 (m, 4H), δ 6.82 - 6.95 (m, 1H), δ 7.21 - 7.55 (m, 7H). MS (APCI): 393.5 (M+1).

### EXAMPLE 41

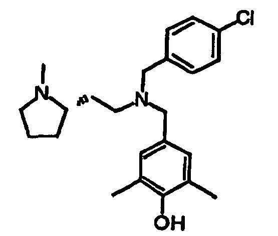

¹HNMR(CD₃OD):δ 1.41.1.59 (m,1H), δ 1.82-2.04(m,10H), δ 2.28-2.45(m,1H), δ 2.77(s,3H), δ 2.93 -3.10(m, 3H), δ 3.11-3.21(m, 1H), δ 3.49-3.61 (m, 1H), δ 4.07-4.39 (m, 4H), δ 6.96 - 7.05 (m, 2H), δ 7.31 - 7.40 (m, 2H), δ 7.41 - 7.52 (m, 2H). MS (APCI): 387.4 (M+1).

### EXAMPLE 42,

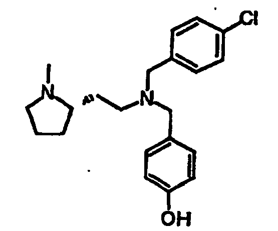

¹H NMR (CD₃OD): δ 1.38 -1.59 (m, 1H), δ 1.78 - 2.13 (m, 4H), δ 2.26 - 2.47 (m, 1H), δ 2.76 (s, 3H), δ 2.90 - 3.10 (m, 3H), δ 3.11 - 3.26 (m, 1H), δ 3.45 - 3.60 (m, 1H), δ 4.13 - 4.38 (m, 4H), δ 6.66 - 6.78 (m, 2H), δ 7.23 - 7.38 (m, 4H), δ 7.42 - 7.53 (m, 2H). MS (APCI): 359.4 (M+1).

### EXAMPLE 43

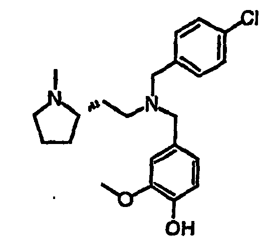

¹H NMR (CD₃OD): δ 1.40 - 1.61 (m, 1H), δ 1.80-2.17 (m, 4H), δ 2.31 - 2.52 (m, 1H), δ 2.80 (s, 3H), δ 2.94 - 3.15 (m, 3H), δ 3.15 - 3.31 (m, 1H), δ 3.57 (m, 1H), δ 3.80 (s, 3H), δ 4.16 - 4.46 (m, 4H), δ 6.66 - 6.83 (m, 1H), δ 6.87 - 7.01 (m, 1H), δ 7.10 - 7.21 (m, 1H), δ 7.30 - 7.45 (m, 2H), δ 7.52 (t, 2H). MS (APCI): 389.4 (m+1).

### EXAMPLE 44

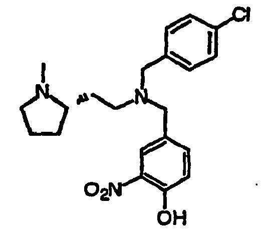

¹H NMR (CD₃OD): δ 1.41 - 1.65 (m, 1H), 81.79 - 2.19 (m, 4H), δ 2.30 - 2.52 (m, 1H), δ 2.75 (s, 3H), δ 2.89 - 3.26 (m, 4H), δ 3.44 - 3.68 (m, 1H), δ 4.19 - 4.46 (m, 4H), δ 7.05 (d, 1H), δ 7.27 - 7.60 (m, 4H), δ 7.63 (d, 1H), δ 8.10 (s, 1H). MS (APCI): 404.1 (M+1).

### Example 45

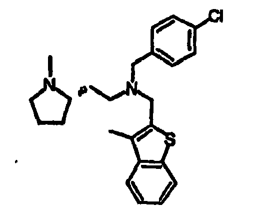

¹H NMR (CDCl₃):δ1.54 -1.75 (m, 1H), δ 1.88 - 226 (m, 2H), δ 2.31 - 2.66 (m, 4H), δ 2.86 (s, 3H), δ3.07 (br-d, 1H),-δ3.20-3.46(m,3H),δ3.63(br-d-1H), δ4.45(s,2H), δ 7.36 - 7.70 (m, 6H), δ 7.74- 7.96 (m, 2H). MS (APCI): 413.1 (M+1).

### EXAMPLE 46

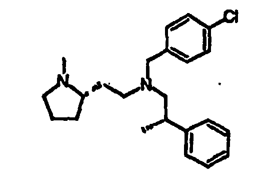

¹HNMR(CD₃OD): δ 1.17(dd, 3H), δ 1.35 - 1.74 (m, 1H), δ 1.80-2.50 (m, 5H), δ 2.69 (s, 1H), δ 2.78 (s, 2H), δ 2.84 - 3.05 (m, 2H), δ 3.07 - 3.22 (m, 2H), δ 3.25 - 3.64 (m, 3H), δ 4.18 - 4.43 (m, 2H), δ 7.07 - 7.52 (m, 5H), δ 7.32 - 7.53 (m, 4H). MS (APCI): 371.7 (M+1).

### EXAMPLE 47

(S)-Di-(4-methylbeazyl)aminoethyl-N-ethylpyrrolidine may be prepared as follows:

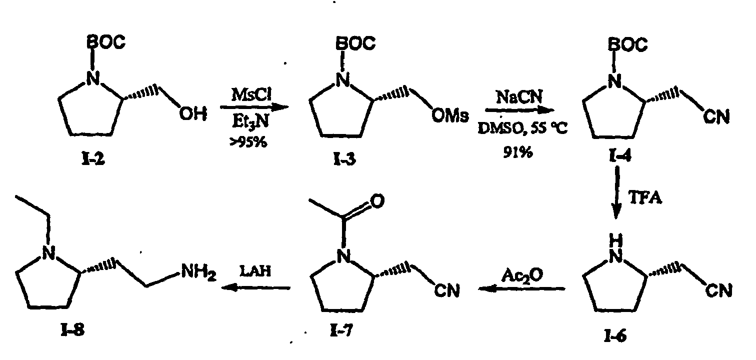

(S)-N-Acetyl-2-cyanomethylpyrrobdme (I-7): N-Boc-2-cyanomethyl-pyrrolidine I-4 (2.10 g) was dissolved in 20 mL of a solution ofTFA in CH₂Cl₂ (voL 1:1) and stirred for 15 minutes. The solvent was concentrated under reduced pressure. The residue was dissolved in 20 mL of THF, treated with 4 ml, of triethylamine (Et₃N) and 2 mL of acetic anhydride, and stirred overnight The resulting mixture was quenched with water and extracted with ethyl acetate. The ethyl acetate phase was dried with Na₂SO₄, filtered and concentrated under reduced pressure. The residue was dissolved in ethyl acetate and filtered through silica gel, which was then washed with more ethyl acetate to provide 1.26 g of the title compound. Confirmation of the identity of the title compounds was confirmed by GC/MS and LC/MS.

(S)2-Aminoethyl-1-ethylpyrrolidine (I-8): Lithium aluminum hydride (30 mL of a 1M solution in THF) was added dropwise to a 0°C solution of N-acetyl-2-cyanomethylpyrrolidine 1-7 (14.1 g, 31.0 mmol) in 35 mL of diethyl ether. After stirring at room temperature for 5 hours, the reaction mixture was heated to reflux for 5 hours, cooled in an ice bath, and quenched with 25% NaOH aqueous solution. The solid formed was filtered and thoroughly washed with THF. The filtrate was dried with Na₂SO₄, filtered and concentrated under reduced pressure to provide 0.65 g of the title compound. It is considered within the ordinary skill of one in the art to prepare other N-substituted analogs of compound 1-8 by straightforward modification of the above method (e.g., using different acylating agents).

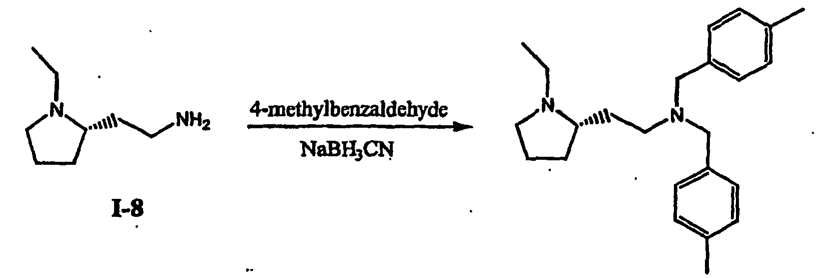

(S)-Di-(4-methylbenzyl)aminoethyl-N-ethylpyrrolidine: Sodium cyanoborohydride (0.75 g, 11.9 mmol) was slowly added to a stirred solution of (S) 2-aminoethyl-N-ethylpyrrolidine (0.21 g,1.5 mmol) and 4-methylbenzaldehyde (0.72 g, 6.0 mmol) in 10 mL of MeOH. The mixture was stirred for 4 hours at room temperature and 4 hours at 50°C, quenched with 2 N HCl, and the solvent was concentrated under reduced pressure. The residue was dissolved in MeOH and filtered, concentrated (223 mg), and purified by HPLC. ¹HNMR (CDCl₃): δ 0.92 - 1.11 (m, 2H), δ 1.26- 1.71 (m, 4H), δ 1.82 - 2.20 (m, 4H), δ 2.24 (s, 6H), δ 2.31 - 2.52 (m, 3H), δ 2.71 - 2.85 (m, 1H), δ 3.07 (m, 1H), δ 3.30 (d, 2H), δ 3.54 (d, 2H), δ 6.97 - 7.26 (m, 8H). MS (APCI): 351.4 (M+1).

Other symmetric compounds encompassed within the scope of this invention may be prepared using Compound I-8, or using other N-substituted analogs of compound I-8, and commercially available or readily available aldehydes by the methods described in Example 1. Asymmetrically substituted compounds encompassed within the scope of this invention may be prepared using Compound I-8, or using other N-substituted analogs of compound I-8, and commercially available or readily available aldehydes by the methods of Examples 13 and 19.

### EXAMPLE 48

(S)-Di-(benzyl)-2-aminomethyl-N-methylpyrrolidine may be prepared according to the following:

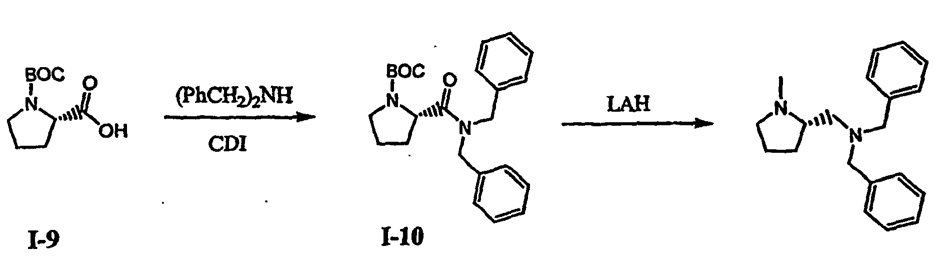

(S)-Di-(benzyl)-2-ammocarbonyl-N-methylpyrrolidine (I-10): To a solution of N-Boc-2-proline (I-9, 2.15 g, 10 mmol) and dibenzylamine (2.17 g, 11 mmol) in 20 mL of THF, was added 1,1-carbonyldiimidazole (CDI; 1.94 g, 12 mmol), and the reaction mixture was stirred for 48 hours. After solvent evaporation, the residue was purified on a silica gel column, using hexane/EtOAc (4:1) first, then 2:1, to provide 3.20 g of the title compound (I-10).

(S)-Di-(benzyl)-2-aminomethyl-N-methylpyrrolidine (D-1): Intermediate I-10 (3.2 g) was dissolved in 50 mL of dry ether, and 50 mL of 1.0 M lithium aluminum hydride solution in THF was added slowly at room temperature, followed by reflux for 14 hours under N₂ atmosphere. The reaction cooled in an ice bath and quenched with 25% aqueous NaOH. The solid formed was filtered out and washed with THF thoroughly. The filtrate was dried with Na₂SO₄ and solvent was concentrated under reduced pressure. The residue was purified on a silica gel column, using chloroform/MeOH (95:5), to provide 0.81 g of the title compound, for a two-step yield of 28%. ¹HNMR (CDCl₃): δ 1.32 - 1.S6 (m, 1H), δ 1.58 - 1.76 (m, 2H), δ 1.92 - 2.05 (m, 1H), δ 2.08 (q, 1H), δ 2.32 (s, 3H), δ 2.32 - 2.47 (m, 2H), δ 2.60 (dd, 1H), δ 2.99 (m, 1H), δ 3.43 (d, 2H), δ 3.69 (d, 2H), δ 7.08 - 7.56 (m, 10H). MS (APCI): 295.1 (M+1).

### BIOCHEMICAL AND BIOLOGICAL ASSAYS

Cells and Membrane Preparation: HEK 293 cells stably expressing human 5-HT_{7B} (h5-HT_{7b}) receptors were grown in Dulbecco's Modified Eagle's Medium (DMEM; Gibco) without sodium pyruvate and containing 4.5 g/L glucose, L-glutamine/penicillin-streptomycin (Gemini), 10% fetal bovine serum and 250 mg/l of the antibiotic, G418 (Geneticin) as previously described (Jasper, J.R., Kosaka, A., To, Z.P., Chang, D.J. and Eglen, R.M. (1997) Cloning, expression and pharmacology of a truncated splice variant of the human 5-ht₇ receptor (h5-ht_{7b}). Br. J. Pharmacol. 122(1):126-132.). Cell pellets were homogenized in approximately 50 mL of homogenization buffer (buffer A) containing: 50 mM Tris (pH 7.4), 2 mM EOTA, 0.32 M sucrose, 10 µM PMSF, 1 µg/mL leupeptin, 5 µg/mL Pepstatin A, and 5 µg/mL aprotinin using an UltraTurax homogenizer (Tekmar Company, Cincinnati, OH) at 80% maximum setting three times for 10 sec. Cell pellets were centrifuged at 4°C at 1,500 x g for 10 min in a Beckman GS-6R centrifuge. Pellets were resuspended in buffer A, homogenized and centrifuged as described above. Pooled supernatants were transferred to centrifuge bottles and centrifuged at 4°C at 20,000 x g for 30 min in a Beckman J2-HS centrifuge. Cell pellets were resuspended in buffer A and were centrifuged at 4°C at 20,000 x g for 30 min. Cell pellets were resuspended in buffer A and stored at -70°C in aliquots of 2.5 mg/mL total membrane protein. Total membrane protein was assessed utilizing a BCA kit (Pierce; Rockford, IL). Membranes containing human 5-HT₁ₐ or 5-HT₂ₐ receptors expressed in CHO K1 cells were prepared as described above. Membranes bearing human D_{2S} dopamine (hD_{2S}-DA) receptors expressed in A9 L cells and human 5-HT₆ (h5-HT₆) receptors expressed in HEK-293 cells were purchased from Receptor Biology, Inc. (Beltsville, Maryland) and were utilized according to the suggested guidelines provided by the manufacturer.

Radioligand Binding Assays: For 5-HT₇ saturation binding experiments, HEK-293 cell membranes expressing h5-HT₇ receptors (5-10 µg membrane protein/well) were incubated in duplicate with [³H]5-CT (approximately 0.2 nM) in binding assay buffer containing: 50 mM HEPES (pH 7.4), 0.5 mM EDTA,10 mM MgCl₂, 10 µM pargyline to inhibit monoamine oxidase activity, and 0.1 % sodium ascorbate, in a final volume of 200 µL in 96-well polypropylene plates for 2 hours at 37°C. Nonspecific binding was determined by incubating membranes with 1 µM 5-HT. All radioligand binding assays were stopped by rapid filtration onto 96-well GF/C filter plates (Packard) soaked in 0.1% polyethylenimine. Filters were washed three times with ice-cold phosphate-buffered saline (PBS) wash buffer containing 50 mM NaPO₄ (pH 7.4), 0.9% NaCl, 2 mM MgCl₂ and 0.02% NaN₃. The filters were then counted using liquid scintillation in a Packard Topcount scintillation counter.

Competition binding to the other receptor types was assayed in a similar fashion, under conditions summarized in Table 1 below.

| Table I. Competition Radioligand Binding Assay Conditions | | | | | | |
|---|---|---|---|---|---|---|
| Assay | [Radioligand] nM | Nonspecific binding defined | [Membrane] µg/well | Time/ Temp⁺ | Assay Volume (mL) | Binding Buffer⁺⁺ |
| h5-HT_{7b} | [³H]5-CT 0.2-0.3 | 1 µM 5-HT | 5-10 | 2 hr @ 37°C | 0.2 | A |
| h5-HT₂ₐ | [³H]Ketanserin 0.5-1.0 | 10 nM Clozapine | 10-20 | 1hr @ 37°C | 0.2 | B |
| h5-HT₆ | [³H]LSD 2.0-3.0 | 100 nM Methiothepin | 25-30 | 1hr@ RT | 0.2 | C |
| H5-HT₁ₐ | [³H]5-CT 0.2-0.3 | 10 nM 5-CT | 5-10 | 1br @ RT | 0.2 | D |
| hD₂ₛ DA | [³H]Spiperone 0.08-0.15 | 1 µM Haloperidol | 25-35 | 2 hr @ RT | 2.0 | E |

| | | | | | | |
|---|---|---|---|---|---|---|
| ⁺RT = room temperature | | | | | | |
| ⁺⁺Buffer compositions: | | | | | | |
| A: 50 mM HEPES (pH 7.4), 0.5 mM EDTA, 10 mM MgCl₂, 10 µM pargyline, 0.1% sodium ascorbate. | | | | | | |
| B: 50 mM Tris (pH 7.4), 0.1% sodium ascorbate | | | | | | |
| C: 50 mM Tris (pH 7.4), 10 mM MgCl₂, 0.5 mM EDTA | | | | | | |
| D: 50 mM Tris (pH 7.4), 10 mM MgCl2, 0.1 % sodium ascorbate | | | | | | |
| E: 50 mM Tris (pH 7.4), 5 mM MgCl₂, 1 mM EDTA, 0.1% sodium ascorbate | | | | | | |

Cyclic AMP Determination: The ability of various compounds to increase basal or to inhibit 5HT-stimulated cAMP formation in HEK-293 cells expressing h5-HT_{7b} receptors was assessed utilizing adenylyl cyclase flashplates custom synthesized by New England Nuclear (NEN). Cells (approximately 50,000 cells/well) were incubated with compounds in a total volume of 100 µl on 96-well adenylyl cyclase flashplates (NEN) for 20 minutes at room temperature with compounds to assess for agonist activity. To assess for antagonist activity, cells were incubated for 1 hr at room temperature with test compounds and then were stimulated for 20 min with 5-HT (10 nM). 100 µl of detection mix containing ¹²⁵I-cAMP was added to quench reactions according to the manufacturer's instructions. Plates were counted on a Packard TopCount after approximately two hours. Control dose-response curves to 5-HT were generated for each plate. Cyclic AMP levels were determined from standard curves generated to non-radioactive cAMP standards (10 nM-1 µM). By this method, all of the Formula I compounds acted as antagonists at 5-HT₇ receptors.

Data Analysis: Radioligand binding experiments were analyzed with Prism™ (GraphPad, San Diego, CA), a computer graphics and statistics program. IC₅₀ values and Hill slopes for compounds were generated by nonlinear regression using Prism™. Values for Kᵢ were calculated from IC₅₀ values by the Cheng and Prussof equation (Cheng, Y. and Prusoff, W.H., (1973), "Relationship between the inhibition constant (Kᵢ) and the concentration of inhibitor which causes 50 per cent inhibition (I₅₀) of an enzymatic reaction." Biochemical Pharmacol. 22:3099-3108).

Biochemical ActivityThe Formula compounds were assayed for binding activity vs. 5-HT₁, 5-HT_{2A}, 5-HT₆, and 5-HT₇ receptor subtypes, as well as dopamine D₂ receptors. Data are summarized in Table 2 below, where entries are blank in cases where the particular assay was not performed.

| Table 2. Kᵢ (nM) for 5-HT and Dopamine Receptors | | | | | |
|---|---|---|---|---|---|
| Compound of Example No. | 5-HT_{1A} | 5-HT_{2A} | 5-HT₆ | 5-HT₇ | D2 |
| 1 | 3500 | 500 | 640 | 9.7 | >2500 |
| 2 | - | 2000 | 2640 | 49 | >2500 |
| 3 | - | 20 | 41 | 9.9. | 110 |
| 4 | 540 | 39 | 42 | 4.7 | 91 |
| 5 | 1780 | 29 | 53 | 80 | 200 |
| 6 | 2850 | 17 | 63 | 2.1 | 221 |
| 7 | - | - | - | 3200 | - |
| 8 | - | - | - | 5100 | - |
| 9 | - | - | - | 1000 | - |
| 10 | - | - | - | - | - |
| 11 | - | - | 74 | 24 | 360 |
| 12 | >4000 | 74 | 210 | 2.7 | 1580 |
| 13 | >4000 | 540 | 880 | 8.8 | >2500 |
| 14 | - | - | - | - | |
| 15 | >4000 | 44 | 39 | 8.3 | 220 |
| 16 | >4000 | 16 | 47 | 28 | 160 |
| 17 | >4000 | 20 | 72 | 31 | 410 |
| 18 | >4000 | 87 | 99 | 45 | 570 |
| 19 | - | - | 20 | 18 | 400 |
| 20 | - | - | - | 150 | - |
| 21 | >4000 | 73 | 91 | 19 | 270 |
| 22 | >4000 | 69 | 33 | 23 | 1010 |
| 23 | >4000 | 60 | 53 | 20 | 660 |
| 24 | - | 130 | 45 | 66 | 1210 |
| 25 | - | 11 | 71 | 36 | 850 |
| 26 | >4000 | 51 | 35 | 56 | 780 |
| 27 | 825 | 61 | 315 | 8.6 | 1750 |
| 28 | >4000 | 3470 | 2710 | 17 | >2500 |
| 29 | >4000 | 200 | 1580 | 49 | 2530 |
| 30 | >4000 | 160 | 375 | 66 | 2300 |
| 31 | - | - | - | 200 | - |
| 32 | 3920 | 85 | 49 | 0.91 | 420 |
| 33 | >4000 | 23 | 132 | 6.5 | 1110 |
| 34 | >4000 | 39 | 83 | 6.6 | 1480 |
| 35 | >4000 | 88 | 54 | 21 | >2500 |
| 36 | - | - | - | 180 | - |
| 37 | - | - | - | 260 | - |
| 38 | >4000 | 27 | 38 | 12 | 250 |
| 39 | 3850 | 42 | 350 | 80 | 1110 |
| 40 | >4000 | 180 | 110 | 2.7 | 1010 |
| 41 | 3070 | 233 | 71 | 4.2 | >2500 |
| 42 | >4000 | 148 | 177 | 1.9 | 1310 |
| 43 | >4000 | 180 | 280 | 3.2 | >2500 |
| 44 | - | - | - | 200 | - |
| 45 | >4000 | 76 | 310 | 27 | 1110 |
| 46 | - | - | - | 161 | - |
| 47 | 610 | 56 | 300 | 63 | 380 |
| 48 | - | - | - | 170 | - |

Biological Activity: The biological activity of the inventive compounds is determined by assays that have been devised to serve as animal models for various human medical conditions. Many such assays are known to skilled practitioners. Useful assays include: the prokinetic assay, which is an in vivo method of determining the extent the test compound affects the rate of gastric emptying of a test meal in rats; the anxiolytic behavior assay, which measures the extent to which the test compound can ameliorate the symptoms of natural anxiety in mice when exposed to a novel, brightly lighted environment; the withdrawal anxiety assay, which measures the extent to which the test compound can ameliorate the symptoms in mice caused by withdrawal from addictive substances by measuring the extent the drug affects the anxiety that occurs in mice after chronically treating with an addictive substance and then abruptly ceasing the treatments; and the cognitive enhancement assay, which measures the extent the test compound can alleviate the cognitive deficit induced in rats by administration of atropine to the rats. These assays are described in U.S. Patent No. 5,763,468, the disclosure of which is hereby incorporated herein by reference.

## Claims

1. A compound of formula: 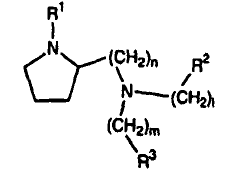
where:
I, m, and n are independently 1 or 2;
R¹ is C₁-C₆ alkyl;
R² and R³ are independently selected from substituted or unsubstituted aryl, heteroaryl, arylalkyl, heteroarylalkyl, and cycloalkenyl, provided that when R¹ is ethyl and 1, m and n are 1, R² and R³ are not both unsubstituted phenyl;
and pharmaceutically acceptable salts or solvates, thereof with the exception of the following compounds:
- (3-bromo-benzyl)-(1-ethyl-pyrrolidin-2-ylmethyl)-naphthalen-1-ylmethyl-amine,
- (5-bromo-2-methoxy-benzyl)-(1-ethyl-pyrrolidin-2-ylmethyl)-naphthalen-1-ylmethyl-amine,
- (1-ethyl-pyrrolidin-2-ylmethyl)-naphthalen-2-yl-methyl-naphthalen-1-ylmethyl-amine.

2. The compound according to claim 1, wherein 1 and m are 1.

3. The compound according to claim 1, wherein n is 2.

4. The compound according to claim 1, wherein R¹ is selected from methyl or ethyl.

5. The compound according to claim 1, wherein R² and R³ are independently selected from substituted or unsubstituted benzyl, methyldibenzylfuranyl, cyclohexenyl, fluorenyl, phenyl, naphthyl, furanyl, benzofuranyl and benzothienyl.

6. The compound according to claim 5, wherein said substituted benzyl, methyldibenzylfuranyl, cyclohexenyl, fluorenyl, phenyl, naphthyl, furanyl, benzofuranyl and benzothienyl is substituted by one or more substituents including C₁-C₆ alkyl substituted or unsubstituted aryl, arylalkyl, heteroarylalkyl, cycloalkyl, heterocycloalkyl, heteroaryl, halo, hydroxyl, alkoxy, aryloxy, cycloalkoxy, heteroaryloxy, nitro, alkylthio, arylthio and aminocarboxyl.

7. The compound according to claim 1, having formula:

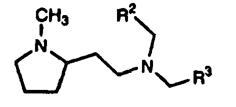

8. The compound according to claim 5, having formula:

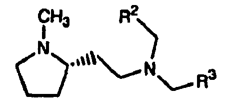

9. A compound according to claim 1 having the formula: 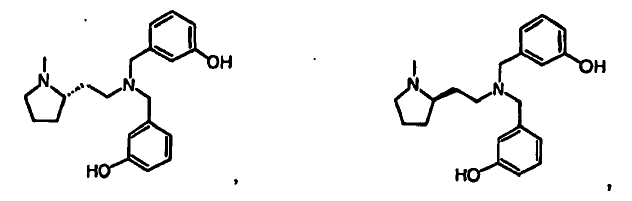 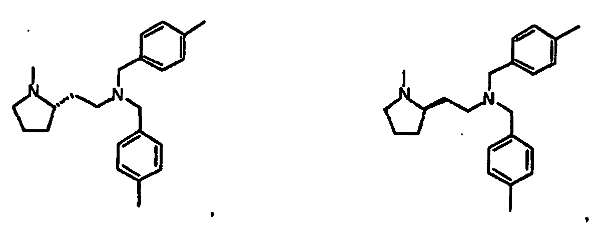 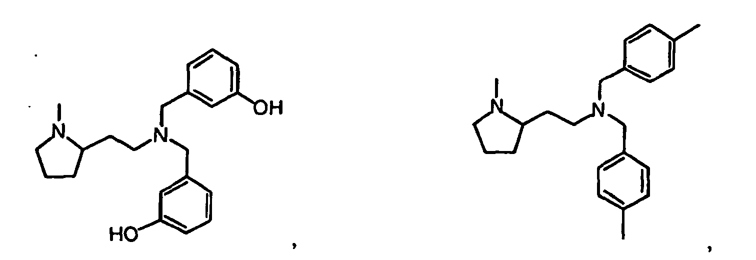 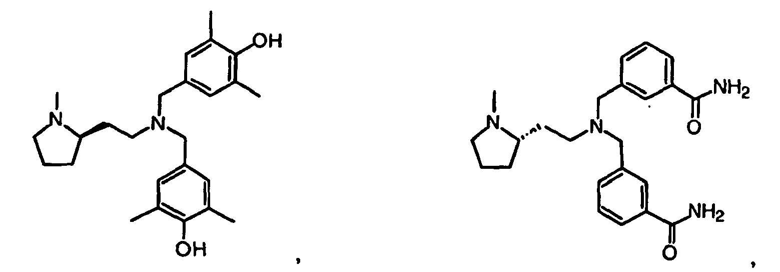 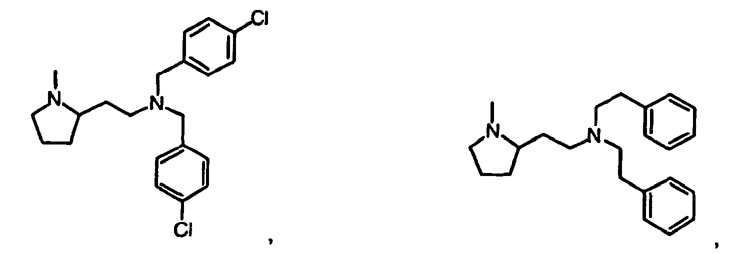 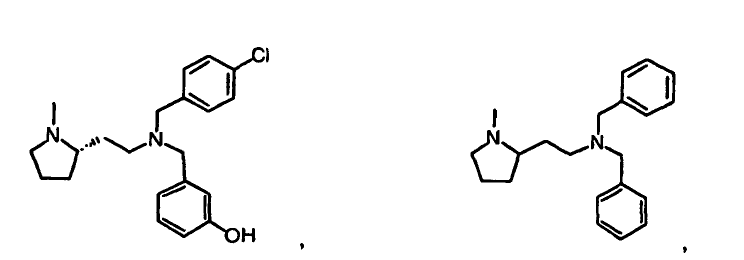 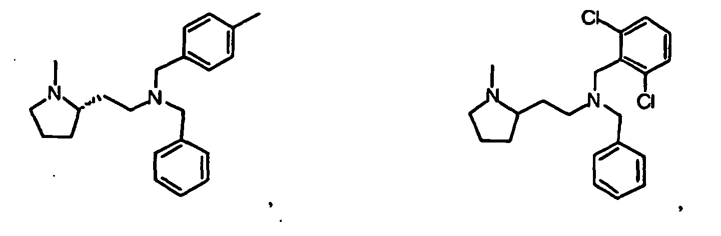 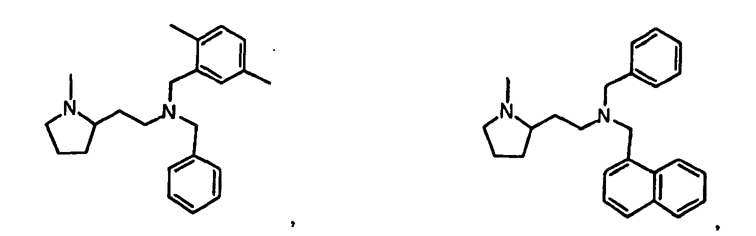 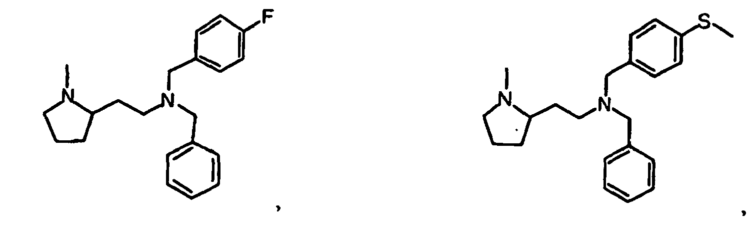 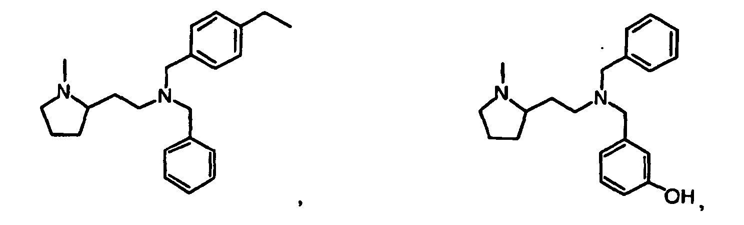 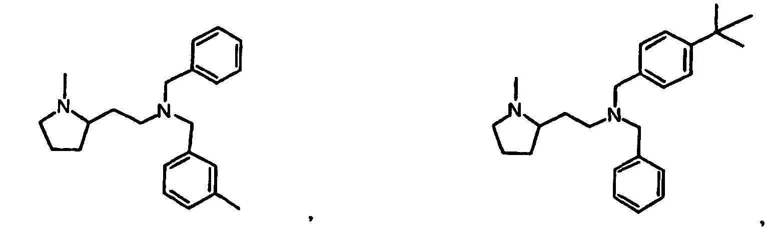 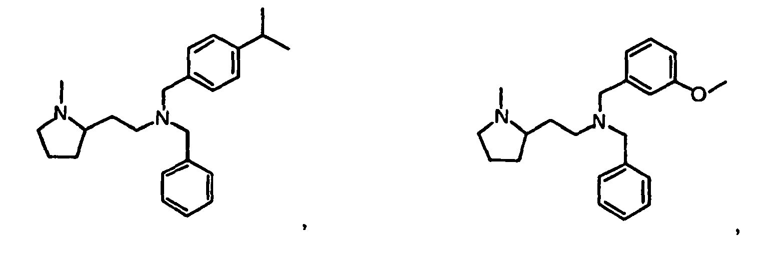 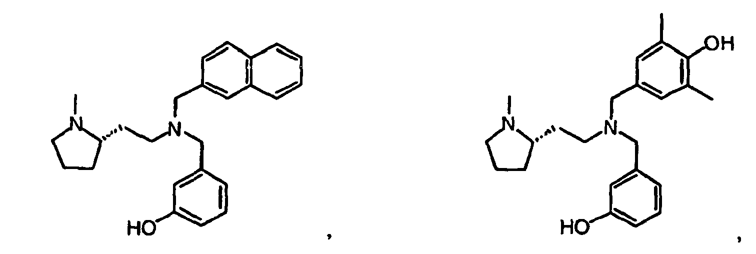 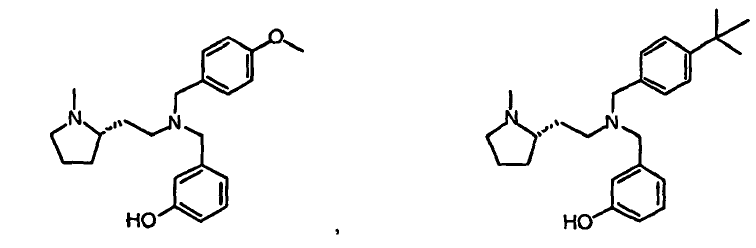 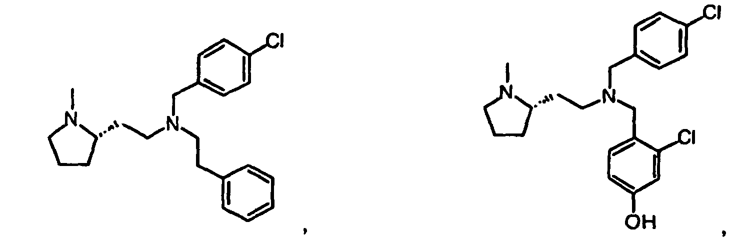 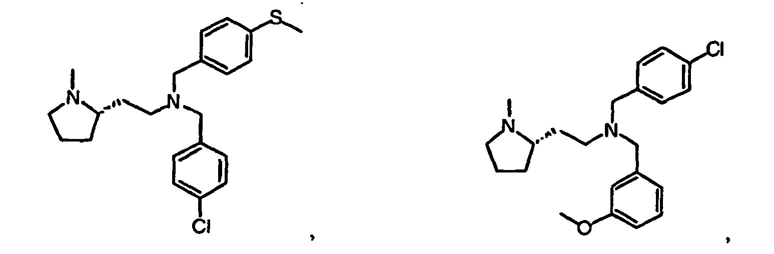 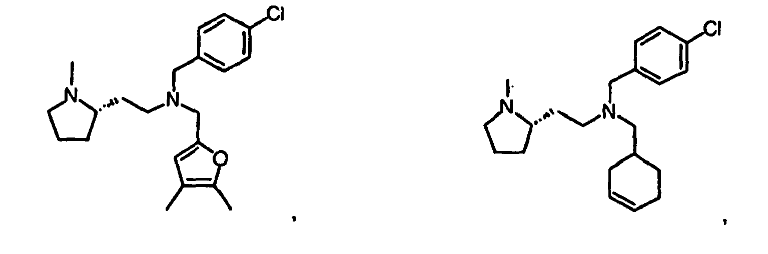 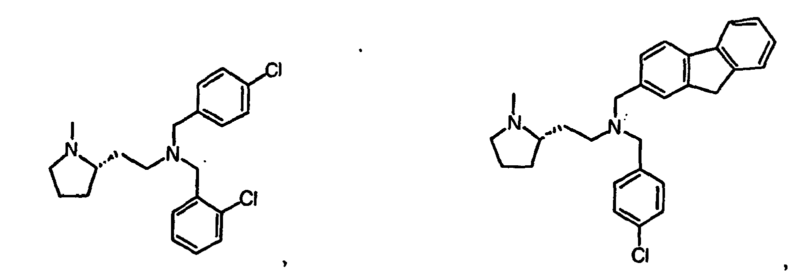 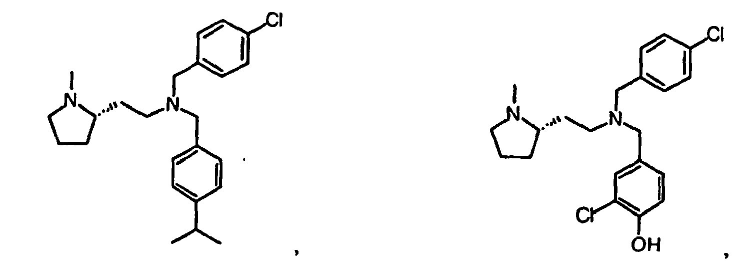 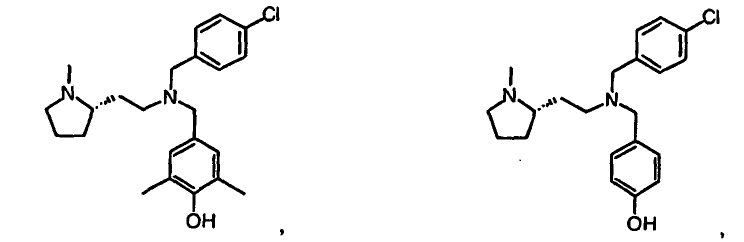 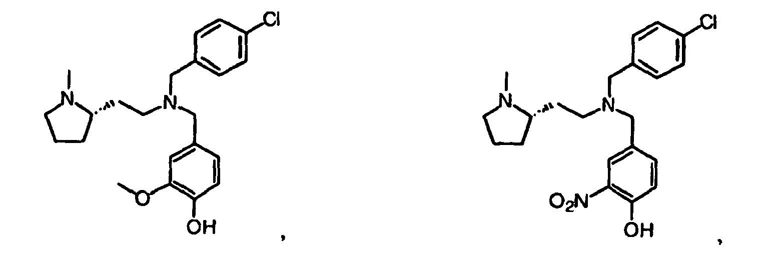 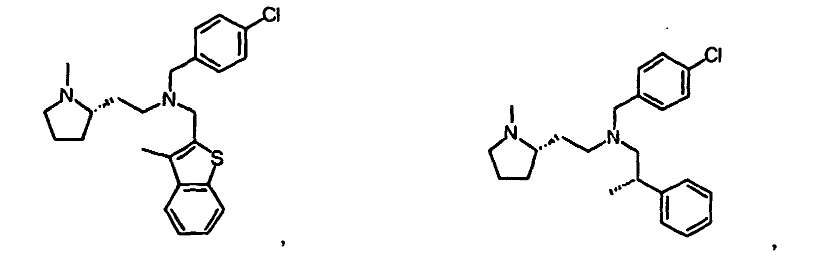 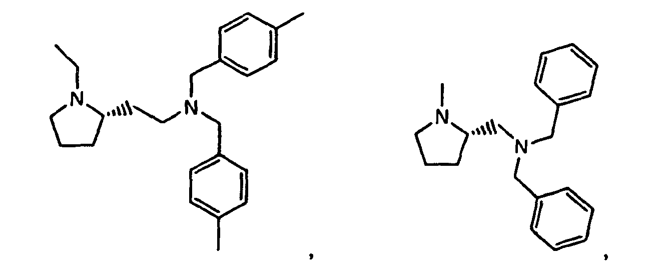 and pharmaceutically acceptable salts or solvates thereof.

10. The compound according to claim 9 having the formula:

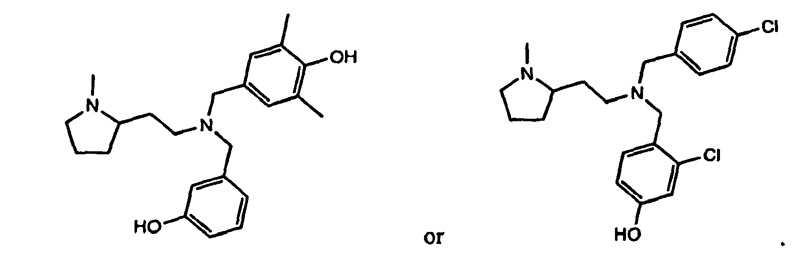

11. A pharmaceutical composition comprising an effective amount of a compound according to claim 1, or a pharmaceutically acceptable salt or solvate thereof.

12. A pharmaceutical composition comprising an effective amount of a compound according to claim 9, or a pharmaceutically acceptable salt or solvate thereof.

13. A method of preparing a compound of formula: 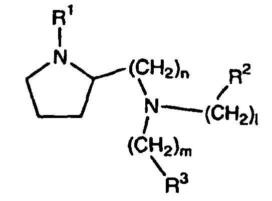
wherein:
l, m, and n are independently 1 or 2;
R¹ is C₁-C₆ alkyl;
R² and R³ are independently selected from aryl, heteroaryl, arylalkyl, heteroarylalkyl, and cycloalkenyl, each optionally substituted by one or more substituents, provided that when R¹ is ethyl and 1, m and n are all 1, R² and R³ are not both unsubstituted phenyl;
comprising the steps of:
(a) coupling under reducing conditions a compound of formula: 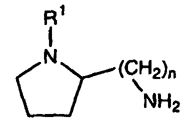
with one equivalent of a compound of formula R²-(CH₂)pCHO, wherein p is (1-1), and
(b) coupling under reducing conditions the coupled product of step (a) with one equivalent of a compound of formula R³-(CH₂)_{q}CHO, wherein q is (m-1).

14. A compound having the formula: 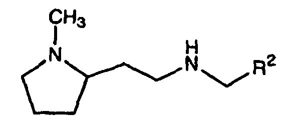
wherein R² is selected from aryl, heteroaryl, arylalkyl, heteroarylalkyl and cycloalkenyl, each optionally substituted by one or more substituents, with the exception of the following compounds:
- (2,5-dimethyl-1-phenyl-1H-pyrrol-3-ylmethyl)-[2-(1-methyl-pyrrolidin-2-yl)-ethyl]-amine, and,
- benzyl-[2-(1-methyl-pyrrolidin-2-yl)-ethyl]-amine.

15. The compound according to claim 14, having formula:

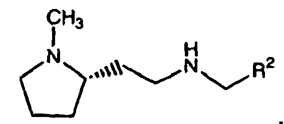

16. A compound according to claim 14 having the formula: 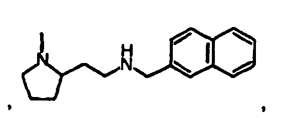 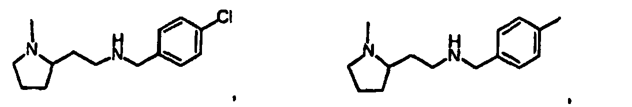 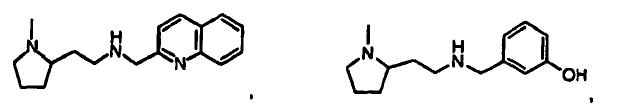 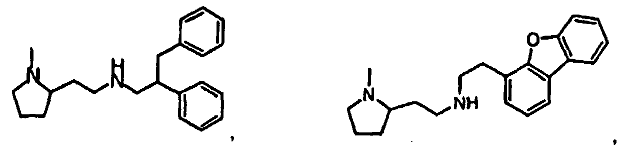 and pharmaceutically acceptable salts or solvates, active thereof.

17. A compound according to claim 1 as a medicament.

18. Use of a compound of formula 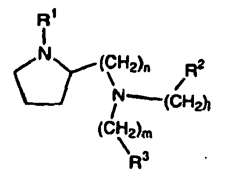
where:
1, m, and n are independently 1 or 2;
R¹ is C₁-C₆ alkyl;
R² and R³ are independently selected from substituted or unsubstituted aryl, heteroaryl, arylalkyl, heteroarylalkyl, and cycloalkenyl, provided that when R¹ is ethyl and l, m and n are I, R² and R³ are not both unsubstituted phenyl;
and pharmaceutically acceptable salts or solvates thereof, for the manufacture of a medicament for the treatment of a disease selected from pain, schizophrenia. depression and sleep disorders.

19. Use according to claim 18 wherein the disease is pain.

20. Use according to claim 18 wherein the disease is schizophrenia.

21. Use according to claim 18 wherein the disease is depression.

22. Use according to claim 18 wherein the disease is sleep disorders.

## Patentansprüche

1. Verbindung der Formel: 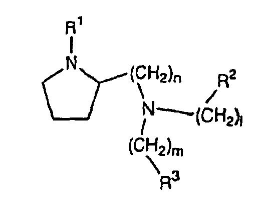
worin:
1, m und n unabhängig 1 oder 2 sind;
R¹ C₁-C₆-Alkyl ist;
R² und R³ unabhängig ausgewählt sind aus substituiertem oder unsubstituiertem Aryl, Heteroaryl, Arylalkyl, Heteroarylalkyl und Cycloalkenyl, mit der Maßgabe, dass, wenn R¹ Ethyl ist und 1, m und n 1 sind, R² und R³ nicht beide unsubstituiertes Phenyl sind;
und pharmazeutisch annehmbare Salze oder Solvate, mit Ausnahme der folgenden Verbindungen:
- (3-Brombenzyl)-(1-ethylpyrrolidin-2-ylmethyl)-naphthalin-1-ylmethylamin,
- (5-Brom-2-methoxybenzyl)-(1-ethylpyrrolidin-2-ylmethyl)naphthalin-1-ylmethylamin,
- (1-Ethylpyrrolidin-2-ylmethyl)naphthalin-2-ylmethyl-naphthalin-1-ylmethylamin.

2. Verbindung nach Anspruch 1, wobei 1 und m 1 sind.

3. Verbindung nach Anspruch 1, wobei n 2 ist.

4. Verbindung nach Anspruch 1, wobei R¹ aus Methyl und Ethyl ausgewählt ist.

5. Verbindung nach Anspruch 1, wobei R² und R³ unabhängig aus substituiertem oder unsubstituiertem Benzyl, Methyldibenzylfuranyl, Cyclohexenyl, Fluorenyl, Phenyl, Naphthyl, Furanyl, Benzofuranyl und Benzothienyl ausgewählt sind.

6. Verbindung nach Anspruch 5, wobei das substituierte Benzyl, Methyldibenzylfuranyl, Cyclohexenyl, Fluorenyl, Phenyl, Naphthyl, Furanyl, Benzofuranyl und Benzothienyl durch einen Substituenten oder mehrere Substituenten, einschließlich C₁-C₆-Alkyl, substituiertes oder unsubstituiertes Aryl, Arylalkyl, Heteroarylalkyl, Cycloalkyl, Heterocycloalkyl, Heteroaryl, Halogen, Hydroxyl, Alkoxy, Aryloxy, Cycloalkoxy, Heteroaryloxy, Nitro, Alkylthio, Arylthio und Aminocarboxyl, substituiert ist.

7. Verbindung nach Anspruch 1 mit der Formel:

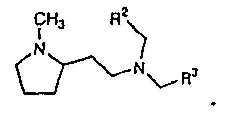

8. Verbindung nach Anspruch 5 mit der Formel:

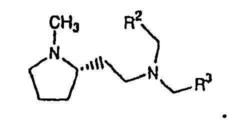

9. Verbindung nach Anspruch 1 mit der Formel: 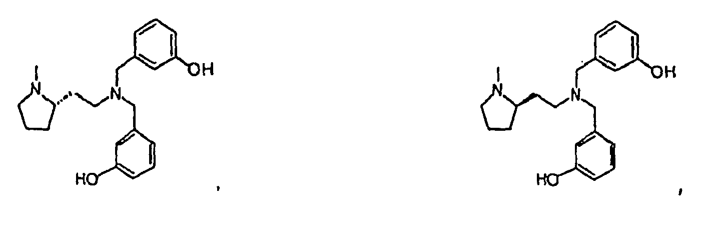 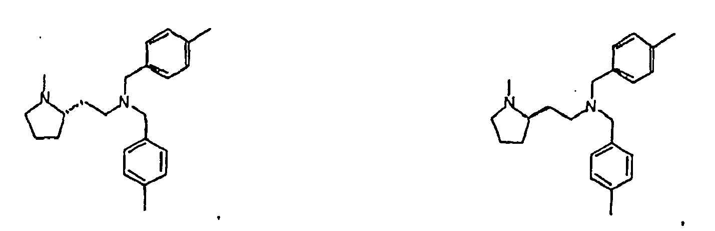 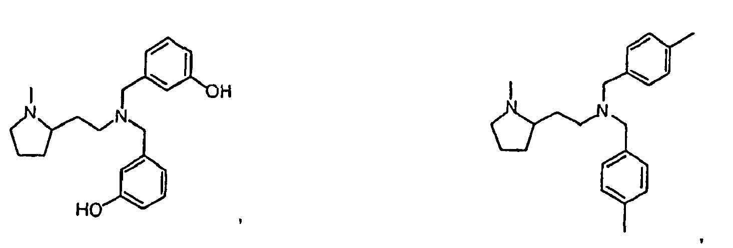 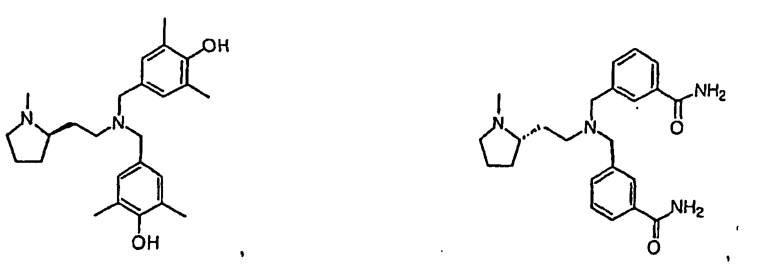 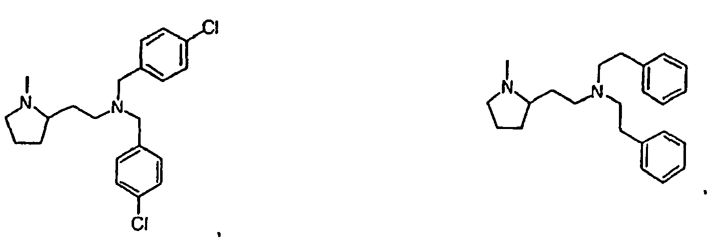 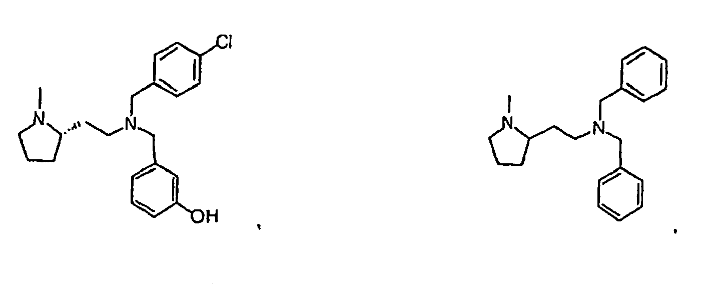 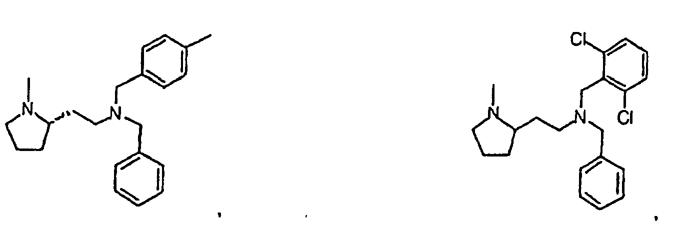 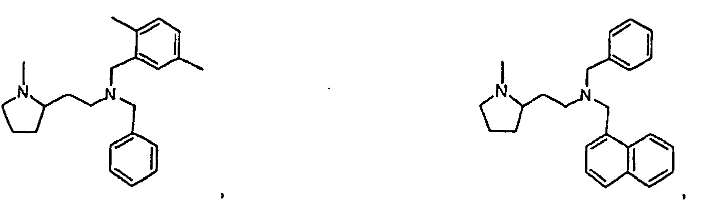 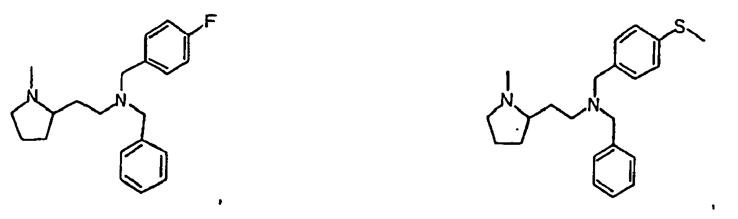 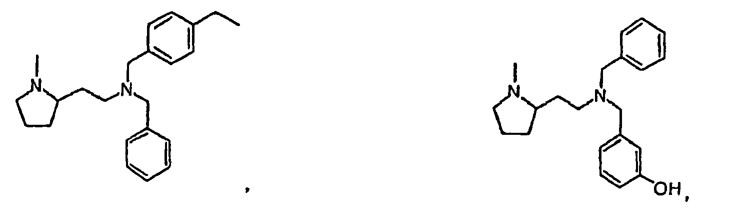 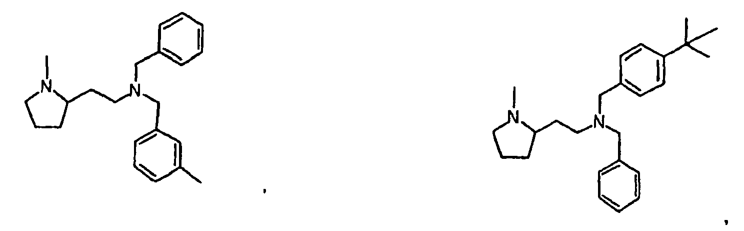 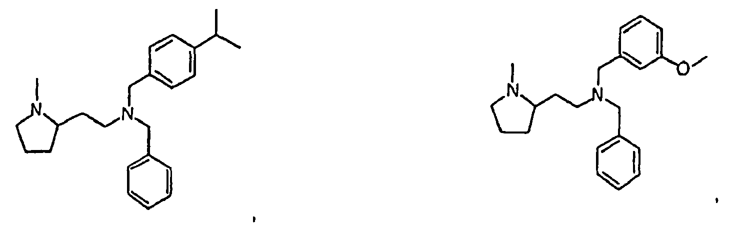 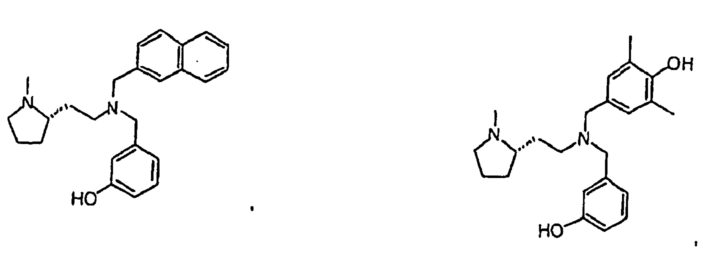 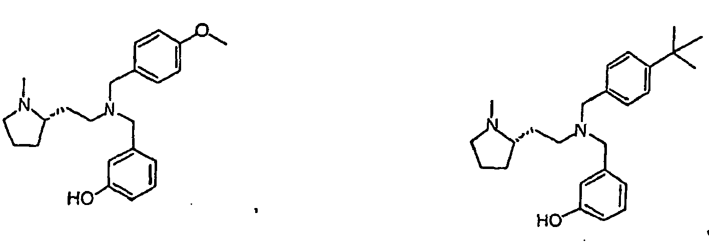 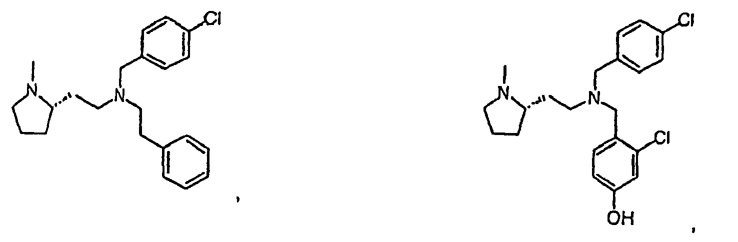 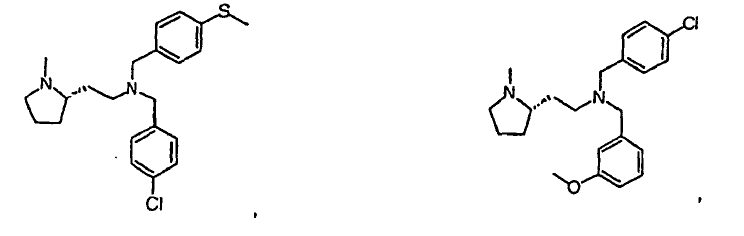 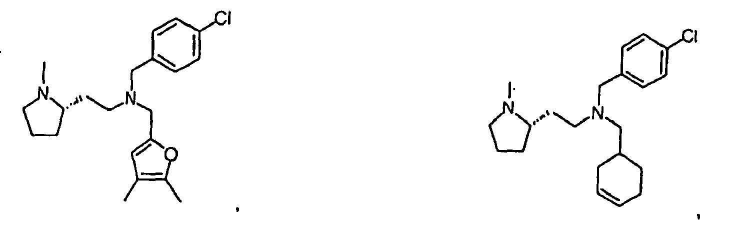 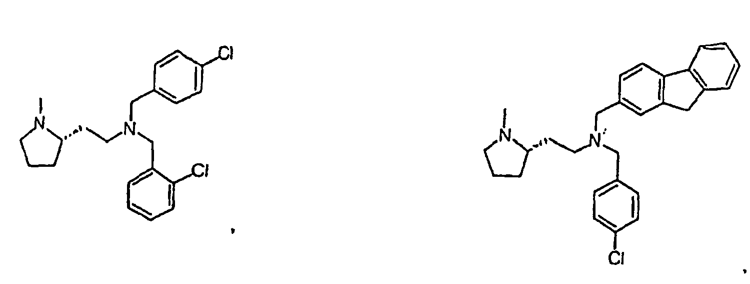 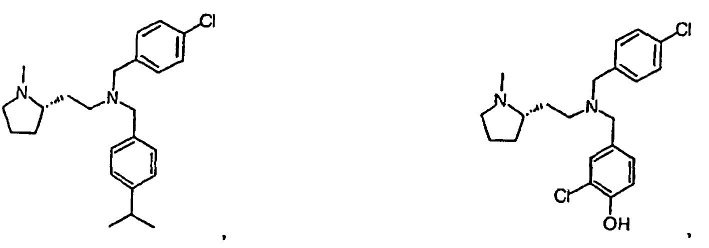 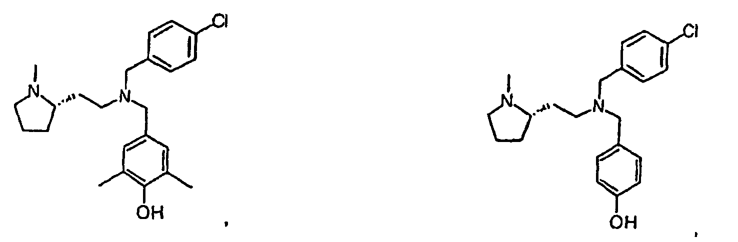 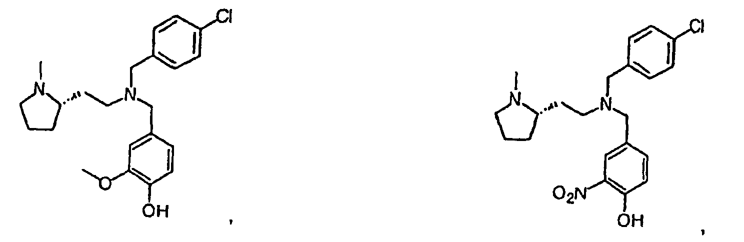 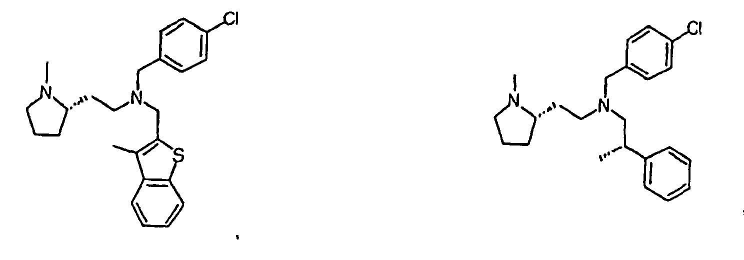 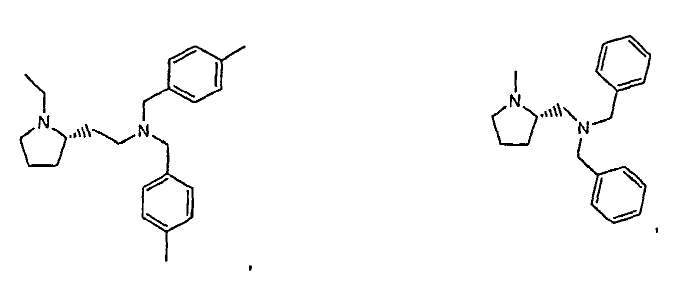 und pharmazeutisch annehmbare Salze oder Solvate davon.

10. Verbindung nach Anspruch 9 mit der Formel:

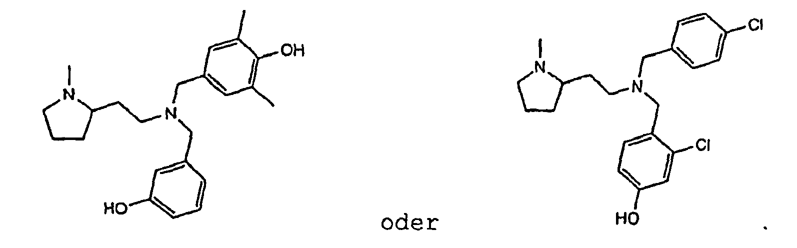

11. Pharmazeutische Zusammensetzung, die eine wirksame Menge einer Verbindung nach Anspruch 1 oder eines pharmazeutisch annehmbaren Salzes oder Solvats davon umfasst.

12. Pharmazeutische Zusammensetzung, die eine wirksame Menge einer Verbindung nach Anspruch 9 oder eines pharmazeutisch annehmbaren Salzes oder Solvats davon umfasst.

13. Verfahren zur Herstellung einer Verbindung der Formel: 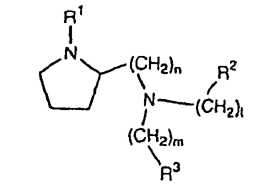
worin:
1, m und n unabhängig 1 oder 2 sind;
R¹ C₁-C₆-Alkyl ist;
R² und R³ unabhängig aus Aryl, Heteroaryl, Arylalkyl, Heteroarylalkyl und Cycloalkenyl, jeweils gegebenenfalls mit einem oder mehreren Substituenten substituiert, ausgewählt sind, mit der Maßgabe, dass, wenn R¹ Ethyl ist und 1, m und n alle 1 sind, R² und R³ nicht beide unsubstituiertes Phenyl sind;
umfassend die Stufen:
(a) Koppeln unter reduzierenden Bedingungen einer Verbindung der Formel: 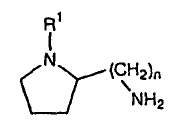
mit einem Äquivalent einer Verbindung der Formel R²- (CH₂)ₚCHO, worin p für (1-1) steht und
(b) Koppeln des gekoppelten Produktes von Stufe (a) unter reduzierenden Bedingungen mit einem Äquivalent einer Verbindung der Formel R³-(CH₂)_{q}CHO, worin q für (m-1) steht.

14. Verbindung der Formel: 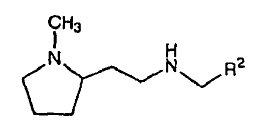
worin R² aus Aryl, Heteroaryl, Arylalkyl, Heteroarylalkyl und Cycloalkenyl, jeweils gegebenenfalls mit einem oder mehreren Substituenten substituiert, ausgewählt ist, mit Ausnahme der folgenden Verbindungen:
- (2,5-Dimethyl-1-phenyl-1H-pyrrol-3-ylmethyl)-[2-(1-methylpyrrolidin-2-yl)ethyl]amin und
- Benzyl-[2-(1-methylpyrrolidin-2-yl)ethyl]amin.

15. Verbindung nach Anspruch 14 mit der Formel:

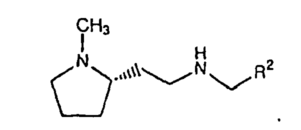

16. Verbindung nach Anspruch 14 mit der Formel: 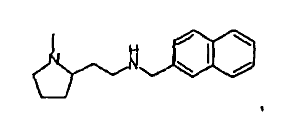 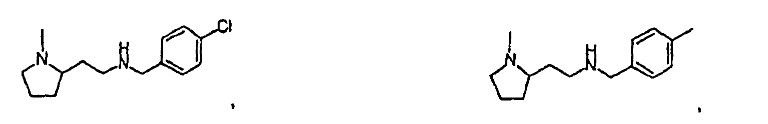 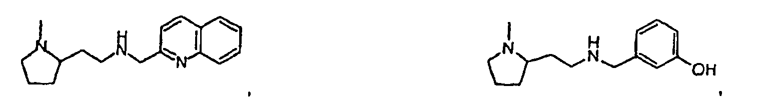 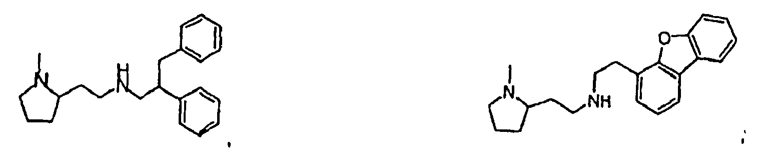 und pharmazeutisch annehmbare Salze davon.

17. Verbindung nach Anspruch 1 als Medikament.

18. Verwendung einer Verbindung der Formel 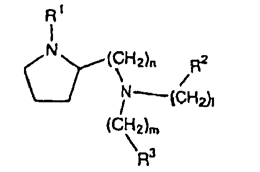
worin:
1, m und n unabhängig 1 oder 2 sind;
R¹ C₁-C₆-Alkyl ist;
R² und _{R}³ unabhängig aus substituiertem oder unsubstituiertem Aryl, Heteroaryl, Arylalkyl, Heteroarylalkyl und Cycloalkenyl ausgewählt sind, mit der Maßgabe, dass, wenn R¹ Ethyl ist und 1, m und n 1 sind, R² und R³ nicht beide unsubstituiertes Phenyl sind;
und pharmazeutisch annehmbare Salze oder Solvate zur Herstellung eines Medikaments zur Behandlung einer Krankheit, ausgewählt aus Schmerzen, Schizophrenie, Depression und Schlafstörungen.

19. Verwendung nach Anspruch 18, wobei die Krankheit Schmerz ist.

20. Verwendung nach Anspruch 18, wobei die Krankheit Schizophrenie ist.

21. Verwendung nach Anspruch 18, wobei die Krankheit Depression ist.

22. Verwendung nach Anspruch 18, wobei die Krankheit Schlafstörungen ist.

## Revendications

1. Composé de formule : 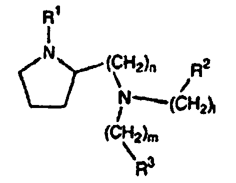
où :
1, m et n valent indépendamment 1 ou 2 ;
R¹ est un groupe alkyle en C₁-C₆ ;
R² et R³ sont indépendamment choisis parmi les groupes aryle, hétéroaryle, arylalkyle, hétéroarylalkyle et cycloalcényle substitués ou non substitués, à condition que lorsque R¹ est un groupe éthyle et 1, m et n valent 1, R² et R³ ne soient pas tous deux des groupes phényle non substitués ;
et les sels ou solvates pharmaceutiquement acceptables de celui-ci, à l'exception des composés suivants :
- (3-bromo-benzyl)-(1-éthyl-pyrrolidin-2-ylméthyl)-naphtalèn-1-ylméthyl-amine,
- (5-bromo-2-méthoxy-benzyl)-(1-éthyl-pyrrolidin-2-ylméthyl)-naphtalèn-1-ylméthyl-amine, et
- (1-éthyl-pyrrolidin-2-ylméthyl)-naphtalèn-2-ylméthyl-naphtalèn-1-ylméthyl-amine.

2. Composé selon la revendication 1, dans lequel 1 et m valent 1.

3. Composé selon la revendication 1, dans lequel n vaut 2.

4. Composé selon la revendication 1, dans lequel R¹ est choisi parmi les groupes méthyle et éthyle.

5. Composé selon la revendication 1, dans lequel R² et R³ sont indépendamment choisis parmi les groupes benzyle, méthyldibenzylfuranyle, cyclohexényle, fluorényle, phényle, naphtyle, furanyle, benzofuranyle et benzothiényle substitués ou non substitués.

6. Composé selon la revendication 5, dans lequel lesdits groupes benzyle, méthyldibenzylfuranyle, cyclohexényle, fluorényle, phényle, naphtyle, furanyle, benzofuranyle et benzothiényle substitués portent un ou plusieurs substituants incluant les groupes alkyle en C₁-C₆, aryle substitués ou non substitués, arylalkyle, hétéroarylalkyle, cycloalkyle, hétérocycloalkyle, hétéroaryle, halogéno, hydroxyle, alcoxy, aryloxy, cycloalcoxy, hétéroaryloxy, nitro, alkylthio, arylthio et aminocarboxyle.

7. Composé selon la revendication 1, possédant la formule :

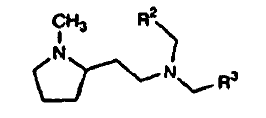

8. Composé selon la revendication 5, possédant la formule :

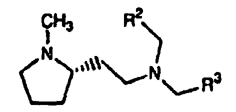

9. Composé selon la revendication 1, possédant la formule : 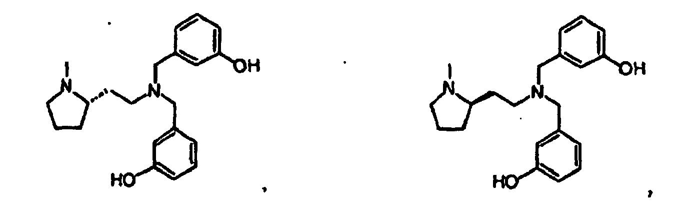 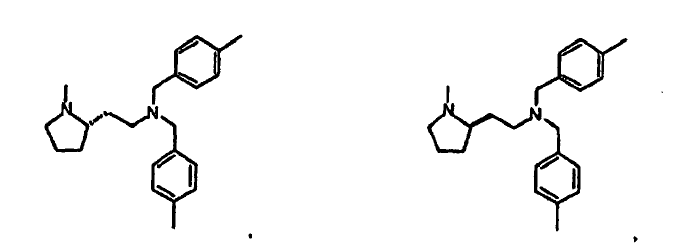 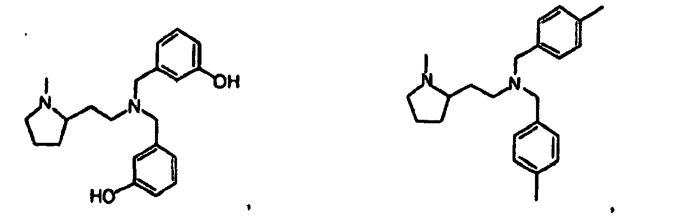 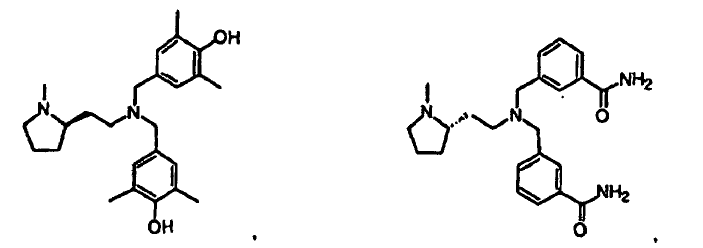 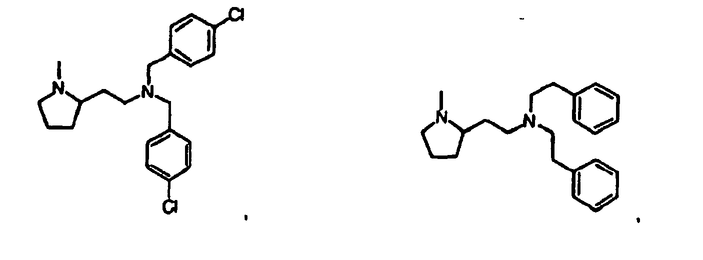 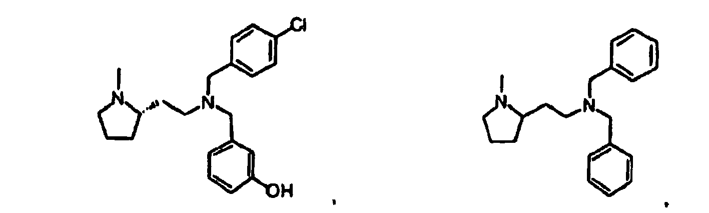 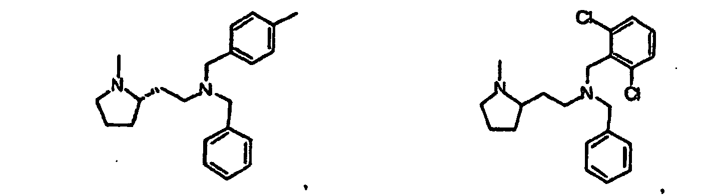 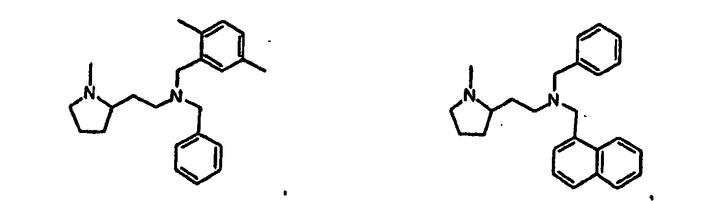 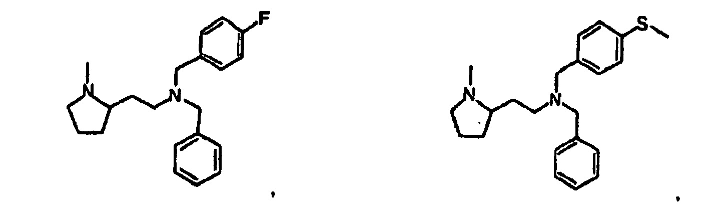 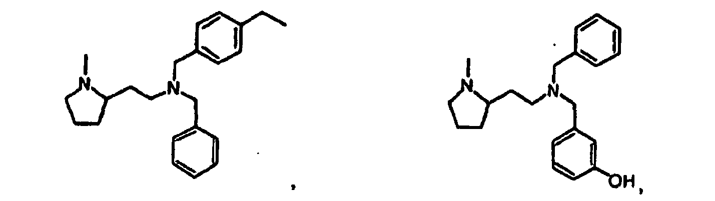 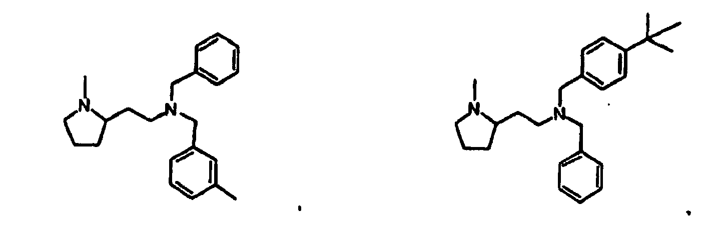 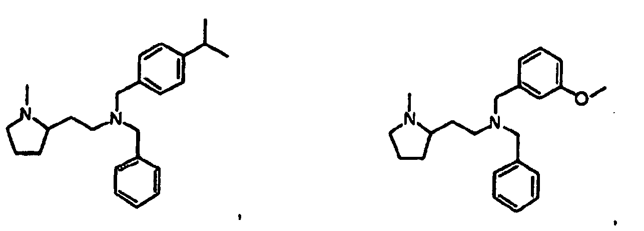 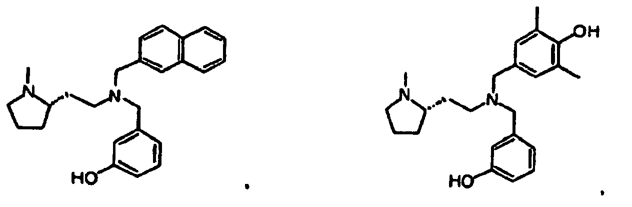 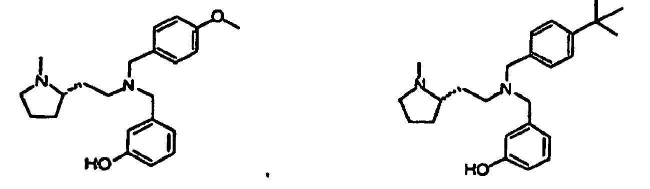 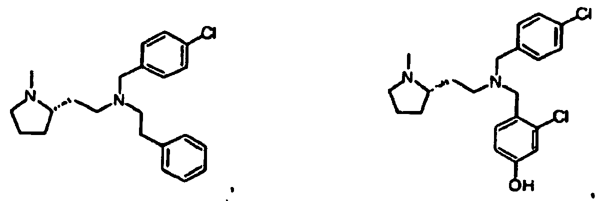 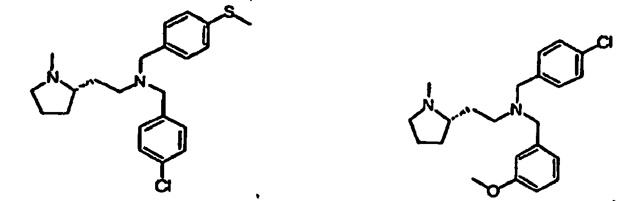 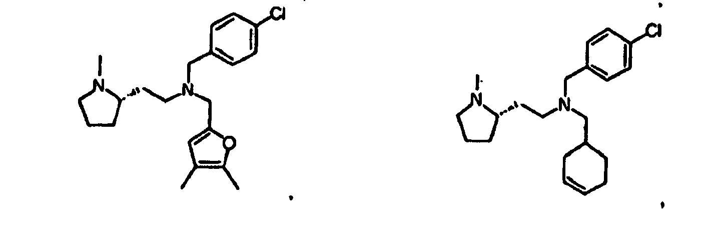 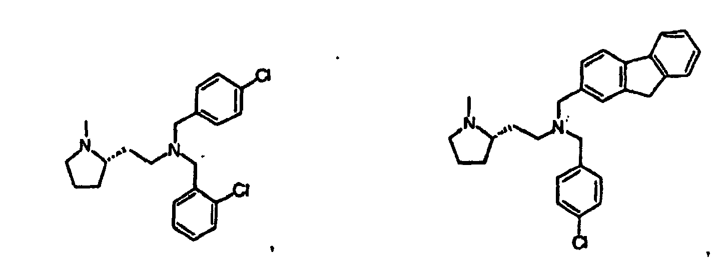 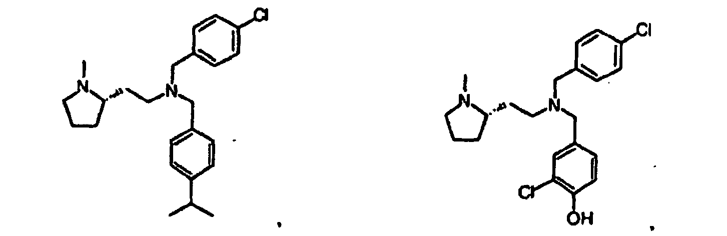 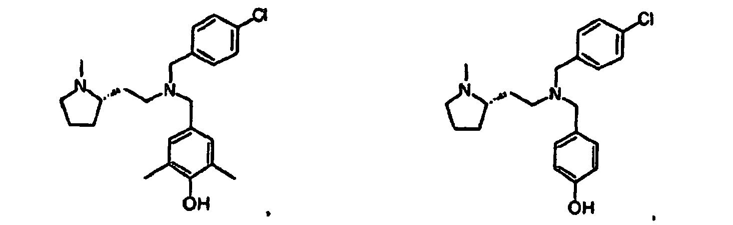 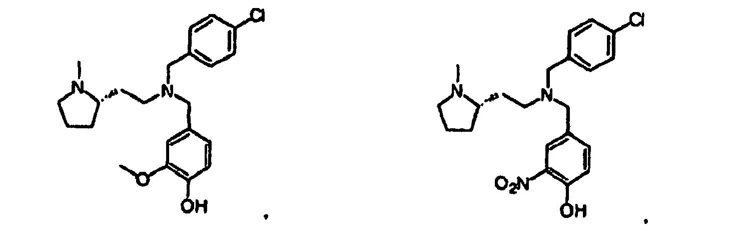 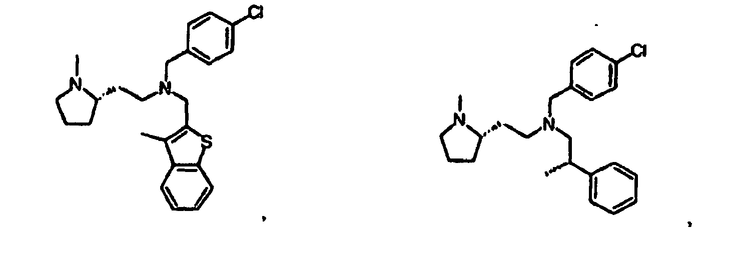 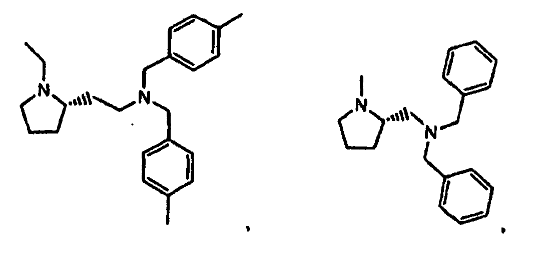 et les sels ou solvates pharmaceutiquement acceptables de celui-ci.

10. Composé selon la revendication 9, possédant la formule :

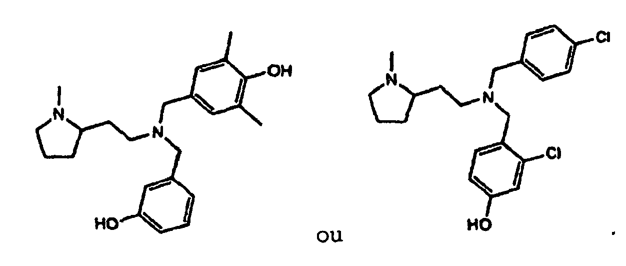

11. Composition pharmaceutique comprenant une quantité efficace d'un composé selon la revendication 1, ou d'un sel ou solvate pharmaceutiquement acceptable de celui-ci.

12. Composition pharmaceutique comprenant une quantité efficace d'un composé selon la revendication 9, ou d'un sel ou solvate pharmaceutiquement acceptable de celui-ci.

13. Procédé de préparation d'un composé de formule : 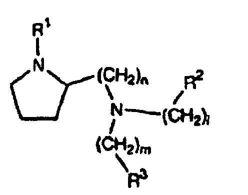
dans laquelle :
1, m et n valent indépendamment 1 ou 2 ;
R¹ est un groupe alkyle en C₁-C₆ ;
R² et R³ sont indépendamment choisis parmi les groupes aryle, hétéroaryle, arylalkyle, hétéroarylalkyle et cycloalcényle, chacun portant éventuellement un ou plusieurs substituants, à condition que lorsque R¹ est un groupe éthyle et 1, m et n valent tous 1, R² et R³ ne soient pas tous deux des groupes phényle non substitués ;
comprenant les étapes de :
(a) couplage dans des conditions réductrices d'un composé de formule : 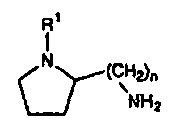
avec un équivalent d'un composé de formule R²-(CH₂)ₚCHO dans laquelle p vaut (1-1), et
(b) couplage dans des conditions réductrices du produit couplé de l'étape (a) avec un équivalent d'un composé de formule R³-(CH₂)_{q}CHO dans laquelle q vaut (m-1).

14. Composé possédant la formule : 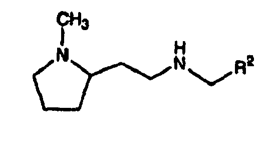 dans laquelle R2 est choisi parmi les groupes aryle, hététoaryle, arylalkyle, hétéroarylalkyle et cycloalcényle, chacun portant éventuellement un ou plusieurs substituants, à l'exception des composés suivants :
- (2,5-diméthyl-1-phényl-1H-pyrrol-3-ylméthyl)-[2-(1-méthyl-pyrrolidin-2-yl)-éthyl]-amine,
- benzyl-[2-(1-méthyl-pyrrolidin-2-yl)-éthyl]-amine.

15. Composé selon la revendication 14, possédant la formule :

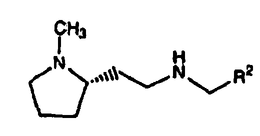

16. Composé selon la revendication 14, possédant la formule : 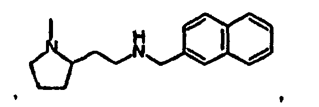 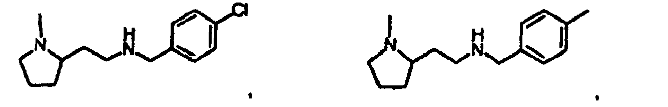 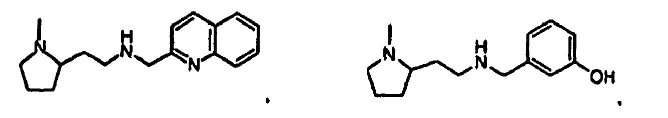 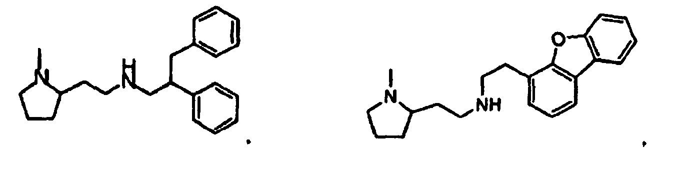 et les sels ou solvates pharmaceutiquement acceptables de celui-ci.

17. Composé selon la revendication 1 en tant que médicament.

18. Utilisation d'un composé de formule : 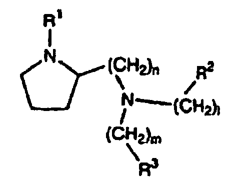
où :
1, m et n valent indépendamment 1 ou 2 ;
R¹ est un groupe alkyle en C₁-C₆ ;
R² et R³ sont indépendamment choisis parmi les groupes aryle, hétéroaryle, arylalkyle, hétéroarylalkyle et cycloalcényle substitués ou non substitués, à condition que lorsque R¹ est un groupe éthyle et 1, m et n valent 1, R² et R³ ne soient pas tous deux des groupes phényle non substitués ;
ou d'un sel ou solvate pharmaceutiquement acceptable de celui-ci, pour la fabrication d'un médicament destiné au traitement d'une maladie choisie parmi la douleur, la schizophrénie, la dépression et les troubles du sommeil.

19. Utilisation selon la revendication 18, dans laquelle la maladie est la douleur.

20. Utilisation selon la revendication 18, dans laquelle la maladie est la schizophrénie.

21. Utilisation selon la revendication 18, dans laquelle la maladie est la dépression.

22. Utilisation selon la revendication 18, dans laquelle la maladie est un trouble du sommeil.
